(19) Europäisches Patentamt
European Patent Office
Office européen des brevets

(11) EP 2 714 030 B1

(12) # EUROPÄISCHE PATENTSCHRIFT

(45) Veröffentlichungstag und Bekanntmachung des Hinweises auf die Patenterteilung:
**12.08.2015 Patentblatt 2015/33**

(21) Anmeldenummer: **12729036.9**

(22) Anmeldetag: **30.05.2012**

(51) Int Cl.:
***A61K 31/295*** (2006.01) ***A61K 31/4453*** (2006.01)
***A61K 31/5375*** (2006.01) ***A61P 7/06*** (2006.01)

(86) Internationale Anmeldenummer:
**PCT/EP2012/060088**

(87) Internationale Veröffentlichungsnummer:
**WO 2012/163938 (06.12.2012 Gazette 2012/49)**

(54) **FE(III)-2,4-DIOXO-1-CARBONYL-KOMPLEXVERBINDUNGEN ZUR VERWENDUNG BEI DER BEHANDLUNG UND DER PROPHYLAXE VON EISENMANGELERSCHEINUNGEN UND EISENMANGELANÄMIEN**

FE(III) 2,4-DIOXO-1-CARBONYL COMPLEXES FOR USE IN THE TREATMENT AND THE PROPHYLAXIS OF IRON DEFICIENCY SYMPTOMS AND IRON DEFICIENCY ANAEMIAS

COMPLEXES DE TYPE 2,4-DIOXO-1-CARBONYLE DE FE(III) POUR LEUR UTILISATION DANS LE TRAITEMENT ET LA PROPHYLAXIE DE CARENCES EN FER ET D'ANEMIES FERRIPRIVES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.05.2011 EP 11168180**

(43) Veröffentlichungstag der Anmeldung:
**09.04.2014 Patentblatt 2014/15**

(73) Patentinhaber: **Vifor (International) AG**
**9001 St. Gallen (CH)**

(72) Erfinder:
- **BARK, Thomas**
  **CH-8050 Zürich (CH)**
- **BUHR, Wilm**
  **78465 Konstanz (DE)**
- **BURCKHARDT, Susanna**
  **CH-8049 Zürich (CH)**
- **BURGERT, Michael**
  **88045 Friedrichshafen (DE)**
- **CANCLINI, Camillo**
  **CH-9000 St. Gallen (CH)**
- **DÜRRENBERGER, Franz**
  **CH-4143 Dornach (CH)**
- **FUNK, Felix**
  **CH-8404 Winterthur (CH)**
- **GEISSER, Peter**
  **CH-9014 St. Gallen (CH)**
- **KALOGERAKIS, Aris**
  **CH-8400 Winterthur (CH)**
- **MAYER, Simona**
  **CH-9055 Bühler (CH)**
- **PHILIPP, Erik**
  **CH-9320 Arbon (CH)**
- **REIM, Stefan**
  **CH-8404 Stadel Winterthur (CH)**
- **SIEBER, Diana**
  **CH-9030 Abtwil (CH)**
- **SCHMITT, Jörg**
  **CH-9425 Thal (CH)**
- **SCHWARZ, Katrin**
  **CH-9000 St. Gallen (CH)**
- **KLOTZ, Jürgen**
  **CH-9042 Speicher (CH)**

(74) Vertreter: **Gille Hrabal**
**Brucknerstrasse 20**
**40593 Düsseldorf (DE)**

(56) Entgegenhaltungen:
**WO-A1-2011/117225**
**JP-A- 4 221 391**
**JP-A- 5 271 157**
**US-A1- 2006 134 227**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 714 030 B1

## Beschreibung

### Einleitung:

Die Erfindung betrifft Eisen(III)-2,4-dioxo-1-carbonyl-Komplexverbindungen mit einen Liganden der Formel (I) und deren sie umfassende pharmazeutische Zusammensetzungen zur Verwendung bzw. Anwendung als Arzneimittel zur Behandlung und/oder zur Prophylaxe von Eisenmangelerscheinungen und Eisenmangelanämien.

### Hintergrund:

Eisen ist ein essentielles Spurenelement für fast alle Lebewesen und ist dabei insbesondere für das Wachstum und die Blutbildung relevant. Die Balance des Eisenhaushaltes wird dabei primär auf der Ebene der Eisenwiedergewinnung aus Hämoglobin von alternden Erythrozyten und der duodenalen Absorption von nahrungsgebundenem Eisen reguliert. Das freigesetzte Eisen wird über den Darm insbesondere durch spezifische Transportsysteme (DMT-1, Ferroportin, Transferrin, Transferrin Rezeptoren) aufgenommen, in den Blutkreislauf transportiert und hierüber in die entsprechenden Gewebe und Organe weitergeleitet.

Das Element Eisen ist im menschlichen Körper unter anderem für den Sauerstofftransport, die Sauerstoffaufnahme, Zellfunktionen, wie den mitochondrialen Elektronentransport und letztlich für den gesamten Energiestoffwechsel von großer Bedeutung.

Der Körper eines Menschen enthält im Durchschnitt 4 bis 5 g Eisen, wobei dies in Enzymen, in Hämoglobin und Myoglobin, sowie als Depot- oder Reserve-Eisen in Form von Ferritin und Hämosiderin vorliegt.

Etwa die Hälfte dieses Eisens ca. 2 g liegt als Häm Eisen gebunden im Hämoglobin der roten Blutkörperchen vor. Da Erythrozyten nur eine begrenzte Lebensdauer aufweisen (75-150 Tage), müssen ständig neue gebildet und alte eliminiert werden (über 2 Mio. Erythrozyten werden pro Sekunde neu gebildet). Diese hohe Neubildungskapazität wird durch Makrophagen erreicht, indem diese die alternden Erythrozyten phagozytotisch aufnehmen, lysieren und so das enthaltene Eisen für den Eisenhaushalt rezyklieren können. Somit wird die täglich für die Erythropoese benötige Eisenmenge von ca. 25 mg grösstenteils bereit gestellt.

Der tägliche Eisenbedarf eines erwachsenen Menschen beträgt zwischen 0,5 und 1,5 mg pro Tag, bei Kleinkindern sowie bei Frauen in der Schwangerschaft liegt der Eisenbedarf bei 2 bis 5 mg pro Tag. Die täglichen Eisenverluste, z.B. durch Abschilferung von Haut- und Epithelzellen ist gering, erhöhte Eisenverluste treten beispielsweise bei der Menstruationsblutung bei Frauen auf. Generell können Blutverluste den Eisenhaushalt beträchtlich verringern, da pro 2 ml Blut etwa 1 mg Eisen verloren gehen. Der normale tägliche Eisenverlust von ca. 1 mg wird üblicherweise bei einem erwachsenen, gesunden Menschen über die tägliche Nahrungsaufnahme wieder ersetzt. Der Eisenhaushalt wird über Resorption reguliert, wobei die Resorptionsquote des in der Nahrung vorhandenen Eisens zwischen 6 und 12 % beträgt, bei Eisenmangel beträgt die Resorptionsquote bis zu 25 %. Die Resorptionsquote wird vom Organismus in Abhängigkeit vom Eisenbedarf und der Eisenspeichergröße reguliert. Dabei nutzt der menschliche Organismus sowohl zweiwertige als auch dreiwertige Eisenionen. Üblicherweise werden Eisen(III)-Verbindungen bei ausreichend saurem pH-Wert im Magen gelöst und damit für die Resorption verfügbar gemacht. Die Resorption des Eisens findet im oberen Dünndarm durch Mukosazellen statt. Dabei wird dreiwertiges nicht Häm Eisen für die Resorption z.B. durch Ferrireduktase (membranständiges, duodenales Cytochrom b) in der Darmzellmembran zunächst zu Fe(II) reduziert, um dann durch das Transportprotein DMT1 (divalent metal transporter 1) in die Darmzellen transportiert werden zu können. Dagegen gelangt Häm Eisen unverändert über die Zellmembran in die Enterozyten. In den Enterozyten wird Eisen entweder als Depot-Eisen in Ferritin gespeichert oder durch das Transportprotein Ferroportin ins Blut abgegeben. In diesem Vorgang spielt Hepcidin eine zentrale Rolle, da es den wesentlichen Regulationsfaktor der Eisenaufnahme darstellt. Das durch das Ferroportin ins Blut transportierte zweiwertige Eisen wird durch Oxidasen (Ceruloplasmin, Hephaestin) in dreiwertiges Eisen überführt, welches dann mittels Transferrin an die relevanten Stellen im Organismus transportiert wird (s. zum Beispiel: "Balancing acts: molecular control of mammalian iron metabolism". M.W. Hentze, Cell 117,2004,285-297.)

Der Organismus von Säugetieren kann Eisen nicht aktiv ausscheiden. Der Eisenmetabolismus wird im Wesentlichen über die zelluläre Freisetzung von Eisen aus Makrophagen, Hepatocyten und Enterozyten durch das Hepcidin gesteuert.

Ein verringerter Serum-Eisenspiegel führt in krankhaften Fällen zu einem verringerten Gehalt an Hämoglobin, verringerter Erythrozytenproduktion und damit zu einer Anämie.

Äussere Symptome von Anämien beinhalten Müdigkeit, Blässe sowie verringerte Aufmerksamkeitskapazitäten. Die klinischen Symptome einer Anämie beinhalten niedrige Serum-Eisengehalte (Hypoferrämie), geringe Hämoglobingehalte, geringe Hämatokritlevel sowie eine reduzierte Anzahl an roten Blutkörperchen, reduzierte Retikulozyten und erhöhte Werte an löslichen Transferrinrezeptoren.

Eisenmangelerscheinungen oder Eisenanämien werden durch Eisenzufuhr behandelt. Dabei erfolgt die Substitution mit Eisen entweder auf dem oralen Weg oder durch intravenöse Eisengabe. Außerdem können zur Ankurbelung

der Bildung von roten Blutkörperchen auch Erythropoetin und andere Erythropoese-stimulierende Substanzen bei der Behandlung von Anämien eingesetzt werden.

Anämie kann oft auf Mangelernährung bzw. eisenarme Diäten oder unausgewogene, eisenarme Ernährungsgewohnheiten zurück geführt werden. Außerdem treten Anämien durch verringerte bzw. schlechte Eisenabsorption beispielsweise aufgrund von Gastrektomien oder von Erkrankungen wie Crohn's-Disease auf. Auch kann ein Eisenmangel infolge eines erhöhten Blutverlustes z.B. durch eine Verletzung, starke Menstruationsblutung oder Blutspende auftreten. Auch ist ein erhöhter Eisenbedarf in der Wachstumsphase Heranwachsender und Kinder sowie von Schwangeren bekannt. Da ein Eisenmangel nicht nur zu einer verringerten Bildung von roten Blutkörperchen, sondern damit auch zu einer schlechten Versorgung des Organismus mit Sauerstoff führt, was zu den oben genannten Symptomen wie Müdigkeit, Blässe und Konzentrationsschwäche auch gerade bei Heranwachsenden zu langfristigen negativen Auswirkungen auf die kognitive Entwicklung führen kann, ist eine gut wirksame und gut verträgliche Therapie von besonderem Interesse.

Durch die Verwendung der erfindungsgemäßen Fe(III)-Komplexverbindungen besteht die Möglichkeit, Eisenmangelerscheinungen und Eisenmangelanämien durch orale Applikation effektiv zu behandeln, ohne das grosse Nebenwirkungspotential der klassischen Präparate, der Fe(II)-Eisensalze, wie $FeSO_4$, hervorgerufen durch Oxidativen Stress, in Kauf zu nehmen. Somit wird eine schlechte Compliance vermieden, die oft Grund für die mangelnde Beseitigung des Eisenmangelzustands darstellt.

**Stand der Technik:**

Aus dem Stand der Technik sind Eisenkomplex-Verbindungen für die Behandlung von Eisenmangelzuständen bekannt.

Ein sehr grosser Anteil dieser Komplex-Verbindungen besteht aus polymeren Strukturen. Hierbei handelt es sich bei den meisten Komplex-Verbindungen um Eisen-Polysaccharid-Komplexverbindungen (WO20081455586, WO2007062546, WO20040437865, US2003236224, EP150085). Gerade aus diesem Bereich sind schon Medikamente auf dem Markt verfügbar (wie Maltofer, Venofer, Ferinject, Dexferrum, Ferumoxytol).

Ein anderer grosser Bestandteil der Gruppe der polymeren Komplexverbindungen sind die Eisen-Peptid-Komplexverbindungen (CN101481404, EP939083, JP02083400).

In der Literatur sind auch Fe-Komplexverbindungen beschrieben, die sich von Makromolekülen, wie Hämoglobin, Chlorophyll, Curcumin und Heparin strukturell herleiten (US474670, CN1687089, Biometals, 2009,22,701-710).

Ferner sind in der Literatur auch niedermolekulare Fe-Komplexverbindungen beschrieben. Eine Vielzahl dieser Fe-Komplexverbindungen umfasst Carbonsäuren und Aminosäuren als Liganden. Besonders stehen hier Aspartat (US2009035385) und Citrat (EP308362) als Liganden im Vordergrund. In diesem Zusammenhang werden auch Fe-Komplexverbindungen, die derivatisierte Phenylalanin-Reste als Liganden beinhalten beschrieben (ES2044777).

In der Patent-Literatur sind auch Hydroxypyron- und Hydroxypyridon-Fe-Komplexverbindungen beschrieben (EP159194, EP138420, EP107458). Analog hierzu sind auch die entsprechenden 5-Ringsysteme, die Hydroxyfuranon-Fe-Komplexverbindungen beschrieben (WO2006037449).

In der Patent-Literatur sind auch Eisen(III)-2,4-dioxo-1-carbonyl-Komplexverbindungen beschrieben (JP-A-05271157 (JP199270884), JP-A-04221391 (JP199186727)). In diesen Patenten wird die Synthese mehrerer Fe-Komplexe beschrieben, wobei deren Anwendung ausschliesslich auf synthetische Fragestellungen ausgerichtet ist. Hinweise auf mögliche medizinische Anwendungen werden hierbei nicht gegeben.

Die US-A-20060134227 offenbart eine eisenhaltige Nahrungsergänzungs-Zusammensetzung. Als Eisen-Verbindung erwähnt sie unter zahlreichen Verbindungen ein nicht näher spezifiziertes "acetylacetone iron complex salt" (Anspruch 13). Um welche Verbindung es sich dabei genau handelt, bleibt unklar. Es sind verschiedene Eisen(acetylacetonat)-Verbindungen bekannt. Neben $Eisen(II)(acetylacetonat)_2$ (CAS-Nr. 14024-17-0) ist das $Eisen(III)(acetylacetonat)_3$ bekannt, dessen Synthese beispielsweise in CHAUDHURI MIHIR K ET AL: "Novel synthesis of Tris(acetylacetonato)iron (III)", JOURNAL OF THE CHEMICAL SOCIETY. DALTON TRANSACTIONS, ROYAL SOCIETY OF CHEMISTRY, 1. Januar 1983 (1983-01-01), Seiten 839-840, XP009150815, ISSN: 0300-9246 beschrieben wurde. Diese Verbindung erweist sich im Tierversuch jedoch als sehr toxisch (London, J. E.; Smith, D. M. Preliminary toxicological study of ferric acetylacetonate. Report (1983), (LA-9627-MS; Order No. DE83007117), 7 pp. CAN 99:48652 AN 1983:448652 CAPLUS)), weshalb sie offenbar für die Behandlung von Eisenmangelanämien beim Menschen bisher keine Anwendung gefunden hat. Bei den Eisen(acetylacetonat)-Verbindungen handelt es sich um ß-DiketonVerbindungen, die jedoch keine weitere Carbonylgruppe in Nachbarstellung zur β-Diketon-Struktur aufweisen. Es handelt sich also nicht um Eisen(III)-2,4-dioxo-1-carbonyl-Komplexverbindungen mit einem Liganden der Formel (I), wie sie durch die vorliegende Erfindung bereitgestellt werden.

In der WO 2005/074899 A2 werden verschiedene Salze von Chinolin-Verbindungen offenbart, darunter auch Eisensalze. Eine Struktur der Salze wird nicht gezeigt. Die Salzbildung dient der Langzeitstabilisierung besagter Chinolin-Verbindungen, welche den pharmakologisch wirksamen Bestandteil darstellen. Die Chinolin-Verbindungen dienen der

Behandlung von Autoimmun-Erkrankungen (US 6121287). Auch handelt es sich bei den Chinolin-Verbindungen nicht um 2,4-Diox-1-carbonyl-Verbindungen der Formel (I); wie sie erfindungsgemäß als Komplex-Liganden verwendet werden. Auch M FIALLO: "Metal anthracycline complexes as a new class of anthracycline derivatives", (INORGANICA CHIMICA ACTA, Bd. 137, Nr. 1-2, 1. Juli 1987 (1987-07-01), Seiten 119-121, XP55003467, ISSN: 0020-1693, DOI: 10.1016/S0020-1693(00)87129-7) offenbart weder Eisen(III)-2,4-dioxo-1-carbonyl-Komplexverbindungen noch deren Anwendung zur Behandlung von Eisenmangelerscheinungen.

Ausserdem sind in der Literatur Eisen-Cyclopentadienyl-Komplexverbindungen beschrieben (GB842637).

Im weiteren sind in der Literatur auch 1-Hydroxy-4,6-dimethyl-2(1 *H*)-pyrimidinone als Fe(III)-Liganden beschrieben worden (Bull. Chem. Soc. Jpn., 66, 841-841(1993)). Hierbei wird vorgeschlagen diese Verbindungen als Eisenmaskierungsmittel zur Behandlung von Eisenüberladungszuständen einzusetzen.

Ein anderer wichtiger Bestandteil für die Behandlung von Eisenmangelerscheinungen und Eisenmangelanämien stellen die Eisensalze (z.B. Eisen(II)sulfat, Eisen(II)fumarat, Eisen(III)chlorid, Eisen(II)aspartat, Eisen(II)succinat) dar.

Ein grosses Problem dieser Eisensalze stellt ihre teilweise hohe Unverträglichkeit (bis zu 50%) in Form von Übelkeit, Erbrechen, Durchfall aber auch Obstipation und Krämpfe dar. Ausserdem kommt es bei der Verwendung von diesen Eisen(II)-salzen zum Auftreten freier Fe(II)lonen, die die Bildung (u.a. Fenton Reaktion) von reaktiven Sauerstoff Spezies (ROS) katalysieren. Durch diese ROS kommt es zur Beschädigung von DNA, Lipiden, Proteinen und Kohlenhydraten, was weitreichende Auswirkungen auf Zellen, Gewebe und Organe hat. Diese Problematik ist bekannt und wird in der Literatur weitgehend als Ursache für die hohe Unverträglichkeit angesehen und als Oxidativer Stress bezeichnet.

Die Eisen(III)-2,4-dioxo-1-carbonyl-Komplexverbindungen der vorliegenden Erfindung sind im Stand der Technik somit bisher nicht als Arzneimittel zur Verwendung in der Behandlung und/oder zur Prophylaxe von Eisenmangelerscheinungen und Eisenmangelanämien beschrieben worden.

**Aufgabenstellung:**

Die Aufgabe der vorliegenden Erfindung bestand darin, neue therapeutisch wirksame Verbindungen zu entwickeln, die für eine effektive Therapie zur bevorzugt oralen Behandlung von Eisenmangelerscheinungen und Eisenmangelanämien eingesetzt werden können. Dabei sollten diese Eisenkomplexe gleichzeitig deutlich weniger Nebenwirkungen aufzeigen, als die klassisch verwendeten Fe(II)-Salze. Ausserdem sollten diese Eisenkomplexe im Unterschied zu den bekannten polymeren Eisenkomplexverbindungen möglichst eine definierte Struktur (Stöchiometrie) aufweisen und durch einfache Synthesewege darstellbar sein. Des weiteren sollten die Verbindungen bei oraler Verabreichung zu einer hohen Utilisationsrate des Eisens führen, was durch eine gute Wasserlöslichkeit unterstützt wird. Darüber hinaus sollten die Eisen-Verbindungen über eine möglichst geringe Toxizität verfügen und somit in möglichst hohen Dosierungen verabreichbar sein. Dieses Ziel wurde durch die Entwicklung neuartiger Fe(III)-Komplexverbindungen erreicht.

Weiterhin sollten die neuen Eisenkomplexe so beschaffen sein, das sie direkt über die Membran in die Darmzellen aufgenommen werden, um somit ihr komplexgebundenes Eisen direkt an das Ferritin oder an das Transferrin abzugeben oder direkt als intakter Komplex in die Blutbahn zu gelangen. Die neuen Verbindungen sollten aufgrund ihrer Eigenschaften praktisch nicht zum Auftreten von hohen Konzentrationen an freien Eisenionen führen. Denn gerade durch freie Eisenionen kommt es zum Auftreten von ROS, die letztlich für die auftretenden Nebenwirkungen verantwortlich sind.

Um diese Anforderungen erreichen zu können, entwickelten die Erfinder neue Fe(III)-Komplexverbindungen mit nicht zu grossem Molekulargewicht, mittlerer Lipophilie und optimaler Komplexstabilität.

**Beschreibung der Erfindung:**

Die Erfinder fanden überraschend, dass Fe(III)-Komplexverbindungen mit 2,4-Dioxo-1-carbonyl-Liganden der Formel (I) sich für die oben beschriebenen Anforderungen besonders eignen. Es konnte gezeigt werden, dass diese Fe-Komplexverbindungen eine hohe Eisenaufnahme zeigen, wodurch ein schneller Therapieerfolg bei der Behandlung der Eisenmangelanämie erreicht werden kann. Gerade im Vergleich mit Eisensalzen zeigen die erfindungsgemäßen Komplexverbindungen eine schnellere und höhere Utilisation. Ausserdem weisen diese neuen Systeme deutlich geringere Nebenwirkungen als die klassisch verwendeten Eisensalze auf, da es hier nicht in nennenswertem Ausmaß zum Auftreten von freien Eisenionen kommt. Die erfindungsgemäßen Komplexverbindungen zeigen nahezu keinen Oxidativen Stress, da es nicht zur Bildung von freien Radikalen kommt. Somit treten bei diesen Komplexverbindungen deutlich weniger Nebenwirkungen als bei den aus dem Stand der Technik bekannten Fe Salzen auf. Die Komplexverbindungen zeigen in unterschiedlichen pH-Wert-Bereichen gute Stabilitäten und vergleichsweise gute Löslichkeiten. Des Weiteren verfügen die erfindungsgemäßen Eisenkomplex-Verbindungen über eine sehr geringe Toxizität und können somit in hohen Dosierungen ohne Nebenwirkungen verabreicht werden. Die Komplexverbindungen lassen sich gut herstellen

und eignen sich optimal zur Formulierung von Arzneimitteln, insbesondere zur oralen Verabreichung.

Gegenstand der Erfindung sind somit Eisen(III)-2,4-dioxo-1-carbonyl-Komplexverbindungen mit einem Liganden der Formel (I) oder deren pharmazeutisch verträgliche Salze zur Verwendung bzw. Anwendung als Arzneimittel bzw. gleichbedeutend zur Anwendung in einem Verfahren zur therapeutischen Behandlung des menschlichen Körpers in der Behandlung und Prophylaxe von Eisenmangelerscheinungen und Eisenmangelanämien.

Die Eisen(III)-2,4-dioxo-1-carbonyl-Komplex-verbindungen schließen insbesondere solche Verbindungen ein, die folgendes Strukturelement aufweisen:

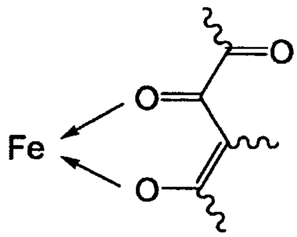

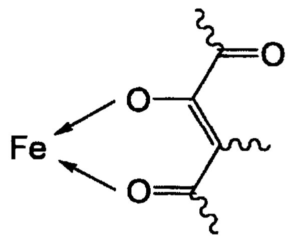

bzw. der mesomeren Grenzformel

worin

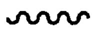

jeweils ein die freie Valenz absättigender Substituent des Liganden ist und die Pfeile jeweils koordinative Bindungen zum Eisenatom darstellen. Die Sauerstoffatome (bzw. die Kohlenstoffatome, an die sie binden) befinden sich also zueinander in 1-, 2- und 4-Position, wobei die Sauerstoffatome in 2- und 4-Position zur 1-Carbonylgruppe an ein Eisenatom binden:

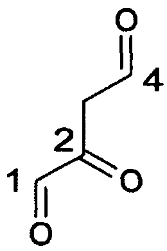

,

wobei der eigentliche Ligand aus dem entsprechenden Enol:

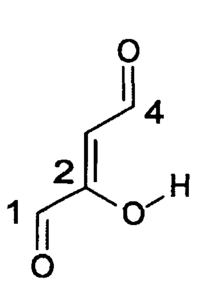

bzw.

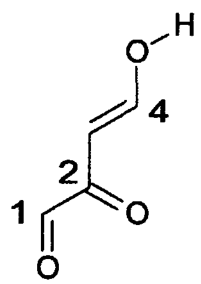

resultiert (die Enolform kann aufgrund der C=C-Doppelbindung in Form von Konfigurationsisomeren (cis/trans bzw. E/Z) vorliegen). Auf die entsprechende Tautomerie wird nachstehend noch ausführlich eingegangen.

Der 2,4-Dioxo-1-carbonyl-Ligand wird nachfolgend auch als 2,4-Dioxocarbonyl-Ligand bezeichnet, weil der Carbonylgruppe beim Grundkörper des Liganden üblicherweise automatisch die 1-Position zugewiesen wird. Im Rahmen der vorliegenden Erfindung werden die Begriffe "2,4-Dioxo-1-carbonyl-Ligand" und "2,4-Dioxocarbonyl-Ligand" also

gleichbedeutend bzw. synonym verwendet.

Der dem 2,4-Dioxo-1-carbonyl-Ligand bzw. 2,4-Dioxocarbonyl-Ligand zugrundeliegende Grundkörper:

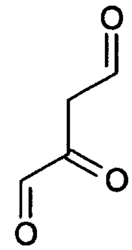

wird nach IUPAC als 2-Oxobutandial bezeichnet (die vorstehend gezeigten Enolformen wären demnach ein 2-Hydroxybut-2-endial bzw. ein 4-Hydroxy-2-oxo-but-3-enal). In diesem Sinne handelt es sich also bei den erfindungsgemäß verwendeten 2,4-Dioxocarbonyl- bzw. 2,4-Dioxo-1-carbonyl-Liganden um (gegebenenfalls substituierte) 2-Oxobutandial-Liganden (Ketoform) bzw. (gegebenenfalls substituierte) 2-Hydroxybut-2-endial-Liganden bzw. (gegebenenfalls substituierte) 4-Hydroxy-2-oxo-but-3-enal-Liganden (Enolformen) (die im Komplex naturgemäß in deprotonierter Form vorliegen (s. die nachstehenden Erläuterungen)).

Klarstellend sei darauf hingewiesen, dass die hier für den 2,4-Dioxo-1-carbonyl-Liganden bzw. 2,4-Dioxocarbonyl-Liganden gewählte Nummerierung der Kohlenstoffpositionen der Carbonylgruppen im Butan-Gerüst als "1,2,4" auf der des Grundkörpers beruht. Abhängig von den Substituenten $R_1$, $R_2$, $R_3$ ist es möglich, dass die IUPAC-Nomenklatur diesen Positionen eine andere Nummerierung zuweist. Ungeachtet dessen handelt es sich dann nach wie vor um 2,4-Dioxo-1-carbonyl-Liganden bzw. 2,4-Dioxocarbonyl-Liganden im Sinne der Erfindung. D.h. der Ligand wird zweifelsfrei gekennzeichnet durch den entsprechenden Butan-Grundkörper bei dem zwei Oxo-Gruppen an benachbarten Kohlenstoffatomen sind und eine OxoGruppe in ß-Stellung zu diesen steht.

Ein 2,4-Dioxocarbonyl-Ligand bzw. 2,4-Dioxo-1-carbonyl-Ligand trägt somit nach Abspaltung eines Protons im Komplex formal eine negative Ladung und das Eisen entsprechend eine positive Ladung je Ligand (d.h., bei drei 2,4-Dioxocarbonyl-Liganden besitzt das Eisen formal die Oxidationsstufe +3 bzw. (III)). Es ist für den Fachmann weiterhin klar, dass es im 2,4-Dioxocarbonyl-Ligand zu einer Delokalisierung der Elektronen kommt.

Außerdem sind auch Eisen(III)-2,4-dioxocarbonyl-Komplexverbindungen umfasst, bei denen der 2,4-Dioxocarbonyl-Ligand eine Brücke zwischen verschiedenen Eisenatomen bildet:

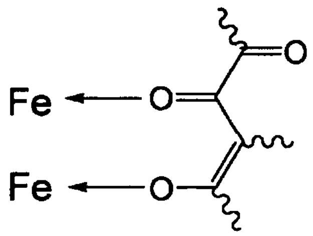

.

Dazu sind insbesondere zweizähnige 2,4-Dioxocarbonyl-Liganden bevorzugt, bei welchen die Bindung zum Eisenatom über die beiden Sauerstoffatome der 2,4-Dioxocarbonyl-Struktureinheit erfolgt. Höherzähnige 2,4-Dioxocarbonyl-Liganden wie drei-, vier-, fünf- oder gar sechs-zähnige 2,4-Dioxocarbonyl-Liganden sind erfindungsgemäß zwar umfasst, jedoch aufgrund ihrer hohen Komplexstabilität (Chelateffekt) weniger bevorzugt, da das Eisen im Körper aufgrund der zu hohen Komplexstabilitäten unter Umständen nicht ausreichend freigesetzt wird. Höherzähnige 2,4-Dioxocarbonyl-Liganden sind insbesondere solche, die neben den beiden Sauerstoffatomen der 2,4-Dioxocarbonylstruktur weitere funktionelle, koordinierende Gruppen aufweisen, wie zum Beispiel eine weitere Carbonylgruppe, analog der allgemeinen Formel (I), oder welche etwa in den unten stehend noch erläuterten Substituentengruppen $R_1$ bis $R_3$ vorliegen. Dabei kann es sich beispielsweise um Sauerstoff- oder Stickstoff-haltige funktionelle Gruppen, wie Hydroxy, Amino oder dergleichen handeln.

Die Eisen(III)-2,4-dioxocarbonyl-Komplexverbindungen schließen insbesondere solche Komplexverbindungen ein, die mindestens einen, bevorzugt zweizähnigen 2,4-Dioxocarbonyl-Liganden aufweisen, der, wie vorstehend gezeigt, an ein oder zwei verschiedene Eisenatome gebunden ist.

Bevorzugt sind Eisen(III)-2,4-dioxocarbonyl-Komplexverbindungen, die ausschließlich, bevorzugt zweizähnige 2,4-Dioxocarbonyl-Liganden aufweisen, welche gleich oder verschieden sein können.

Besonders bevorzugt sind weiterhin Eisen(III)-2,4-dioxocarbonyl-Komplexverbindungen, die ausschließlich die gleichen, bevorzugt zweizähnigen 2,4-Dioxocarbonyl-Liganden aufweisen.

Außerdem umfasst sind jedoch auch solche Komplexverbindungen, die neben dem 2,4-Dioxocarbonyl-Liganden bevorzugt einen oder mehrere (wie zwei oder drei) weitere gleiche oder verschiedene ein- oder mehrzähnige Liganden aufweisen, wie zum Beispiel Carbonsäure- bzw. Carboxylat-Liganden (R-COOH bzw. $RCOO^-$), Alkohol-Liganden (R-OH), wie Kohlenhydrat-Liganden, primäre oder sekundäre Amino-Liganden ($R-NH_2$, R-NHR), Imino-Liganden (R=NH), Oximo-Liganden (R=N-OH), Hydroxyl-Liganden (OH oder $H_2O$), Ether-Liganden, oder Halogen-Liganden. Solche Komplexverbindungen können auch intermediär beim Abbau im Körper also insbesondere in wässriger Lösung auftreten und gegebenenfalls dann auch intermediär koordinativ ungesättigt vorliegen.

In der Eisen(III)-2,4-dioxocarbonyl-Komplexverbindungen beträgt die Koordinationszahl der Eisenatome im Allgemeinen sechs (6), wobei im Allgemeinen eine oktaedrische Anordnung der koordinierenden Atome vorliegt.

Darüber hinaus sind weiterhin ein- oder mehrkernige Eisen(III)-2,4-dioxocarbonyl-Komplexverbindungen umfasst, in denen ein oder mehrere (wie 2, 3 oder 4) Eisenatome vorhanden sind. Bevorzugt sind jedoch einkernige Eisen(III)-2,4-dioxocarbonyl-Komplexverbindungen, in denen ein zentrales Eisenatom vorliegt.

Im allgemeinen können 1-4 Eisenatome und 2-10 Liganden in den Eisen(III)-2,4-dioxocarbonyl -Komplexverbindungen vorliegen. Bevorzugt sind einkernige Eisen(III)-2,4-dioxocarbonyl-Komplexverbindungen mit mindestens einem bevorzugt 3, bevorzugt zweizähnigen 2,4-Dioxocarbonyl-Liganden.

Die Eisen(III)-2,4-Dioxocarbonyl-Komplexverbindungen liegen im Allgemeinen in neutraler Form vor. Es sind jedoch auch salzartige Eisen(III)-2,4-dioxocarbonyl-Komplexverbindungen eingeschlossen, in denen der Komplex eine positive oder negative Ladung aufweist, die insbesondere durch pharmakologisch verträgliche, im wesentlichen nichtkoordinierende Anionen (wie insbesondere Halogenide, wie Chlorid) oder Kationen (wie insbesondere Alkali- oder Erdalkalimetallionen) ausgeglichen wird.

Bevorzugt liegt das Molekulargewicht der erfindungsgemäßen Eisen(III)-2,4-Dioxocarbonyl-Komplexverbindungen bei weniger als 1000 g/mol, noch bevorzugter bei weniger als 800 g/mol (jeweils bestimmt aus der Strukturformel).

Die erfindungsgemäß verwendeten Eisen(III)-Komplexverbindungen enthaltend mindestens einen Liganden der Formel (I):

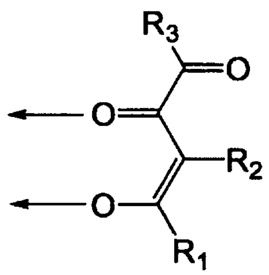

(I)

worin

die Pfeile jeweils eine koordinative Bindung zu einem oder verschiedenen Eisenatomen darstellen,
$R_1$ ausgewählt wird aus der Gruppe, die besteht aus gegebenenfalls substituiertem Alkyl, gegebenenfalls substituiertem Alkoxy und gegebenenfalls substituiertem Alkoxycarbonyl,
$R_2$ ausgewählt wird aus der Gruppe, die besteht aus Wasserstoff, gegebenenfalls substituiertem Alkyl und Halogen, oder
$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ ausgewählt wird aus der Gruppe, die besteht aus gegebenenfalls substituiertem Alkyl, gegebenenfalls substituiertem Alkoxy, gegebenenfalls substituiertem Amino und Hydroxy
oder pharmazeutisch verträgliche Salze davon.

Besonders bevorzugt sind erfindungsgemäß insbesondere Eisen(III)-Komplexverbindungen enthaltend mindestens einen Liganden der Formel (I), worin $R_3$ aus gegebenenfalls substituiertem Amino ausgewählt wird.

Besonders bevorzugt sind erfindungsgemäß weiterhin Eisen(III)-Komplexverbindungen enthaltend mindestens einen Liganden der Formel (I):

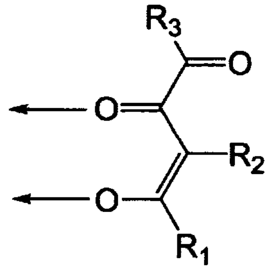

(I)

worin

die Pfeile jeweils eine koordinative Bindung zu einem oder verschiedenen Eisenatomen darstellen,
$R_1$ ausgewählt wird aus der Gruppe, die besteht aus gegebenenfalls substituiertem Alkyl, gegebenenfalls substituiertem Alkoxy oder gegebenenfalls substituiertem Alkoxycarbonyl,
$R_2$ ausgewählt wird aus der Gruppe, die besteht aus Wasserstoff oder gegebenenfalls substituiertem Alkyl, oder
$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ ausgewählt wird aus der Gruppe, die besteht aus gegebenenfalls substituiertem Alkoxy, gegebenenfalls substituiertem Amino und Hydroxy,
oder pharmazeutisch verträgliche Salze davon.

Dabei schließt gegebenenfalls substituiertes Alkyl im Rahmen der gesamten Erfindung insbesondere für die Substituenten $R_1$ bis $R_3$ bevorzugt ein: Geradkettiges oder verzweigtes Alkyl mit 1 bis 8, bevorzugt 1 bis 6, Kohlenstoffatomen, Cycloalkyl mit 3 bis 8, bevorzugt 5 oder 6 Kohlenstoffatomen, oder Alkyl mit 1 bis 4 Kohlenstoffatomen, welches mit Cycloalkyl substituiert ist, wobei diese Alkylgruppen gegebenenfalls substituiert sein können.
Die genannten Alkylgruppen können gegebenenfalls jeweils bevorzugt 1 bis 3 Substituenten tragen.
Diese Substituenten werden bevorzugt aus der Gruppe ausgewählt, die besteht aus: Hydroxy, gegebenenfalls substituiertem Aryl, insbesondere wie nachstehend definiert, gegebenenfalls substituiertem Heteroaryl, insbesondere wie nachstehend definiert, gegebenenfalls substituiertem Alkoxy insbesondere wie nachstehend definiert, gegebenenfalls substituiertem Alkoxycarbonyl, insbesondere wie nachstehend definiert, gegebenenfalls substituiertem Acyl, insbesondere wie nachstehend definiert, Halogen, insbesondere wie nachstehend definiert, gegebenenfalls substituiertem Amino, insbesondere wie nachstehend definiert, gegebenenfalls substituiertem Aminocarbonyl, insbesondere wie nachstehend definiert. Besonders bevorzugte Substituenten von Alkyl sind Hydroxy, Halogen, Alkoxy, Alkoxycarbonyl und Aminocarbonyl.
Halogen schließt hier und im Rahmen der vorliegenden Erfindung Fluor, Chlor, Brom und lod, bevorzugt ist Fluor, ein.
In den vorstehend definierten Alkylgruppen können des weiteren gegebenenfalls ein oder mehrere, bevorzugter 1 bis 3 Kohlenstoffatome durch heteroanaloge Gruppen, die Stickstoff, Sauerstoff oder Schwefel enthalten, ersetzt sein. Dies bedeutet insbesondere, dass beispielsweise eine oder mehrere, bevorzugt 1 bis 3, noch bevorzugter eine (1) Methylengruppe ($-CH_2-$) in den Alkylresten durch -NH-, $-NR_4-$, -O- oder -S- ersetzt sein können, worin $R_4$ gegebenenfalls substituiertes Alkyl, wie vorstehend definiert, bevorzugt gegebenenfalls mit 1 bis 3 Substituenten, wie Fluor, Chlor, Hydroxy, Alkoxy, Alkyl, wie Methyl, Ethyl oder i-Propyl ist.
Beispiele von Alkylresten mit 1 bis 8 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe, eine n-Heptylgruppe, eine 1-Methylhexylgruppe, eine 2-Methylhexylgruppe, eine 3-Methylhexylgruppe, eine 4-Methylhexylgruppe, eine 5-Methylhexylgruppe, eine 1-Ethylpentylgruppe, eine 2-Ethylpentylgruppe, eine 3-Ethylpentylgruppe, eine 4-Ethylpentylgruppe, eine 1,1-Dimethylpentylgruppe, eine 2,2-Dimethylpentylgruppe, eine 3,3-Dimethylpentylgruppe, eine 4,4-Dimethylpentylgruppe, eine 1-Propylbutylgruppe, eine n-Octylgruppe, eine 1-Methylheptylgruppe, eine 2-Methylheptylgruppe, eine 3-Methylheptylgruppe, eine 4-Methylheptylgruppe, eine 5-Methylheptylgruppe, eine 6-Methylheptylgruppe, eine 1-Ethylhexylgruppe, eine 2-Ethylhexylgruppe, eine 3-Ethylhexylgruppe, eine 4-Ethylhexylgruppe, eine 5-Ethylhexylgruppe, eine 1,1-Dimethylhexylgruppe, eine 2,2-Dimethylhexylgruppe, eine 3,3-Dimethylhexylgruppe, eine 4,4-Dimethylhexylgruppe, eine 5,5-Dimethylhexylgruppe, eine 1-Propylpentylgruppe, eine 2-Propylpentylgruppe, usw. Bevorzugt sind solche mit 1 bis 6 Kohlenstoffatomen. Am meisten bevorzugt sind Methyl, Ethyl, n-Propyl und i-Propyl.

Beispiele von Alkylgruppen, die durch Austausch mit einer oder mehreren heteroanalogen Gruppe, wie -O-, -S-, -NH- oder -N($R_4$)- hervorgehen, sind bevorzugt solche, in denen eine oder mehrere Methylengruppen (-$CH_2$-) durch -O- unter Bildung einer Ethergruppe ersetzt sind, wie Methoxymethyl, Ethoxymethyl, 2-Methoxyethyl usw. Daher schließt die Definition von Alkyl auch beispielsweise Alkoxyalkylgruppen, wie nachstehend definiert, ein, welche durch Austausch einer Methylengruppe durch -O- aus den genannten Alkylgruppen hervorgehen. Sind erfindungsgemäß darüber hinaus Alkoxygruppen als Substituenten von Alkyl zugelassen, können auch auf diese Weise mehrere Ethergruppen gebildet werden (wie zum Beispiel eine -$CH_2$-O-$CH_2$-O$CH_3$-Gruppe). Erfindungsgemäß sind somit auch Polyethergruppen von der Definition von Alkyl umfasst. Bevorzugt handelt es sich bei den Alkylgruppen erfindungsgemäß nicht um solche, die aus dem Austausch einer -$CH_2$-Gruppe mit einer oder mehreren heteroanalogen Gruppe, wie -O-, -S-, -NH- oder -N($R_4$)- hervorgehen.

Cycloalkylreste mit 3 bis 8 Kohlenstoffatomen schließen bevorzugt ein: eine Cyclopropylgruppe, eine Cyclobutylgruppe, eine Cyclopentylgruppe, eine Cyclohexylgruppe, eine Cycloheptylgruppe, eine Cyclooctylgruppe usw. ein. Bevorzugt sind eine Cyclopropylgruppe, eine Cyclobutylgruppe, eine Cyclopentylgruppe und eine Cyclohexylgruppe. Die Cycloalkylreste können gegebenenfalls substituiert sein, bevorzugt mit 1 bis 2 Substituenten.

Die Definition der gegebenenfalls substituierten Alkylgruppen schließt auch Alkylgruppen ein, welche durch vorstehend definierte Cycloalkylgruppen substituiert sind, wie beispielsweise Cyclopropylmethyl, Cyclobutylmethyl, Cyclopentylmethyl oder Cyclohexylmethyl.

Heterocyclische Alkylreste sind erfindungsgemäß insbesondere solche, die durch Austausch von Methylen durch heteroanaloge Gruppen aus Cycloalkyl gebildet werden, sind beispielsweise gesättigte 5- oder 6-gliedrige heterocyclische Reste, welche über ein Kohlenstoffatom oder ein Stickstoffatom angebunden sein können, und welche bevorzugt 1 bis 3, bevorzugt 2 Heteroatome wie insbesondere O, N aufweisen können, wie Tetrahydrofuryl, Azetidin-1-yl, substituiertes Azetidinyl, wie 3-Hydroxyazetidin-1-yl, Pyrrolidinyl, wie Pyrrolidin-1-yl, substituiertes Pyrrolidinyl, wie 3-Hydroxypyrrolidin-1-yl, 2-Hydroxypyrrolidin-1-yl 2-Methoxycarbonylpyrrolidin-1-yl, 2-Ethoxycarbonylpyrrolidin-1-yl, 2-Methoxypyrrolidin-1-yl, 2-Ethoxypyrrolidin-1-yl, 3-Methoxycarbonylpyrrolidin-1-yl, 3-Ethoxycarbonylpyrrolidin-1-yl, 3-Methoxypyrrolidin-1-yl, 3-Ethoxypyrrolidin-1-yl, Piperidinyl, wie Piperidin-1-yl, Piperidin-4-yl, substituiertes Piperidinyl, wie 4-Methyl-1-piperidyl, 4-Hydroxy-1-piperidyl, 4-Methoxy-1-piperidyl, 4-Ethoxy-1-piperidyl, 4-Methoxycarbonyl-1-piperidyl, 4-Ethoxycarbonyl-1-piperidyl, 4-Carboxy-1-piperidyl, 4-Acetyl-1-piperidyl, 4-Formyl-1-piperidyl, 1-Methyl-4-piperidyl, 4-Hydroxy-2,2,6,6-tetramethyl-1-piperidyl, 4-(Dimethylamino)-1-piperidyl, 4-(Diethylamino)-1-piperidyl, 4-Amino-1-piperidyl, 2-(Hydroxymethyl)-1-piperidyl, 3-(Hydroxymethyl)-1-piperidyl, 4-(Hydroxymethyl)-1-piperidyl, 2-Hydroxy-1-piperidyl, 3-Hydroxy-1-piperidyl, 4-Hydroxy-1-piperidyl, Morpholin-4-yl, substituiertes Morpholinyl, wie 2,6-Dimethylmorpholin-4-yl, Piperazinyl, wie Piperazin-1-yl, substituiertes Piperazinyl, wie 4-Methylpiperazin-1-yl, 4-Ethylpiperazin-1-yl, 4-Ethoxycarbonylpiperazin-1-yl, 4-Methoxycarbonylpiperazin-1-yl, oder Tetrahydropyranyl, wie Tetrahydropyran-4-yl, und welche gegebenenfalls mit aromatischen Ringen kondensiert sein können, und welche gegebenenfalls substituiert sein können, wie mit 1 bis 2 Substituenten, wie Hydroxy, Halogen, C1-C6-Alkyl etc. Die Definition der gegebenenfalls substituierten Alkylgruppen schließt somit auch Alkylgruppen ein, welche durch vorstehend definierte heterocyclische Gruppen substituiert sind, wie beispielsweise 3-(1-Piperidyl)propyl, 3-Pyrrolidin-1-ylpropyl, 3-Morpholinopropyl, 2-Morpholinoethyl, 2-Tetrahydropyran-4-ylethyl, 3-Tetrahydropyran-4-ylpropyl, 3-(Azetidin-1-yl)propyl etc..

Beispiele eines mit Halogen substituierten linearen oder verzweigten Alkylrestes mit 1 bis 8 Kohlenstoffatomen schließen insbesondere ein:

eine Fluormethylgruppe, eine Difluormethylgruppe, eine Trifluormethylgruppe, eine Chlormethylgruppe, eine Dichlormethylgruppe, eine Trichlormethylgruppe, eine Brommethylgruppe, eine Dibrommethylgruppe, eine Tribrommethylgruppe, eine 1-Fluorethylgruppe, eine 1-Chlorethylgruppe, eine 1-Bromethylgruppe, eine 2-Fluorethylgruppe, eine 2-Chlorethylgruppe, eine 2-Bromethylgruppe, eine 1,2-Difluorethylgruppe, eine 1,2-Dichlorethylgruppe, eine 1,2-Dibromethylgruppe, eine 2,2,2-Trifluorethylgruppe, eine Heptafluorethylgruppe, eine 1-Fluorpropylgruppe, eine 1-Chlorpropylgruppe, eine 1-Brompropylgruppe, eine 2-Fluorpropylgruppe, eine 2-Chlorpropylgruppe, eine 2-Brompropylgruppe, eine 3-Fluorpropylgruppe, eine 3-Chlorpropylgruppe, eine 3-Brompropylgruppe, eine 1,2-Difluorpropylgruppe, eine 1,2-Dichlorpropylgruppe, eine 1,2-Dibrompropylgruppe, eine 2,3-Difluorpropylgruppe, eine 2,3-Dichlorpropylgruppe, eine 2,3-Dibrompropylgruppe, eine 3,3,3-Trifluorpropylgruppe, eine 2,2,3,3,3-Pentafluorpropylgruppe, eine 2-Fluorbutylgruppe, eine 2-Chlorbutylgruppe, eine 2-Brombutylgruppe, eine 4-Fluorbutylgruppe, eine 4-Chlorbutylgruppe, eine 4-Brombutylgruppe, eine 4,4,4-Trifluorbutylgruppe, eine 2,2,3,3,4,4,4-Heptafluorbutylgruppe, eine Perfluorbutylgruppe, eine 2-Fluorpentylgruppe, eine 2-Chlorpentylgruppe, eine 2-Brompentylgruppe, eine 5-Fluorpentylgruppe, eine 5-Chlorpentylgruppe, eine 5-Brompentylgruppe, eine Perfluorpentylgruppe, eine 2-Fluorhexylgruppe, eine 2-Chlorhexylgruppe, eine 2-Bromhexylgruppe, eine 6-Fluorhexylgruppe, eine 6-Chlorhexylgruppe, eine 6-Bromhexylgruppe, eine Perfluorhexylgruppe, eine 2-Fluorheptylgruppe, eine 2-Chlorheptylgruppe, eine 2-Bromheptylgruppe, eine 7-Fluorheptylgruppe, eine 7-Chlorheptylgruppe, eine 7-Bromheptylgruppe, eine Perfluorheptylgruppe, usw.

Beispiele eines mit Hydroxy substituierten Alkylrestes schließen die oben genannten Alkylreste ein, die 1 bis 3 Hydroxylreste aufweisen, wie zum Beispiel Hydroxymethyl, 2-Hydroxyethyl, 3-Hydroxypropyl, 4-Hydroxybutyl, etc.

Gegebenenfalls substituiertes Aryl schließt erfindungsgemäß bevorzugt aromatische Kohlenwasserstoffreste mit 6 bis 14 Kohlenstoffatomen (ohne Heteroatome im Ringsystem) ein, wie beispielweise: Phenyl, Naphthyl, Phenanthrenyl und Anthracenyl. Die genannten aromatischen Reste können gegebenenfalls bevorzugt einen oder mehrere, bevorzugt einen Substituenten aufweisen, insbesondere Halogen, Hydroxy, Alkyl, Alkoxy, jeweils wie vorstehend oder nachstehend erläutert.

Gegebenenfalls substituiertes Aryl schließt erfindungsgemäß weiterhin gegebenenfalls substituiertes Heteroaryl also heteroaromatische Reste ein, wie beispielsweise: Pyridyl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl. 5- oder 6-gliedrige aromatische Heterocyclen wie z.B. Pyridyl, Pyridyl-N-oxid, Pyrimidyl, Pyridazinyl, Furyl und Thienyl sind bevorzugt. Die genannten heteroaromatische Reste können bevorzugt einen oder mehrere, bevorzugt einen Substituenten aufweisen, insbesondere Halogen, Hydroxy, Alkyl, Alkoxy, jeweils wie vorstehend oder nachstehend erläutert. Bevorzugte Beispiele einer mit einer heteroaromatischen Gruppe substituiertes Alkyl (Hetarylalkyl) sind Methyl, Ethyl oder Propyl jeweils substituiert mit einer heteroaromatischen Gruppe, wie Thienylmethyl, Pyridylmethyl etc.

Gegebenenfalls substituiertes Alkoxy (RO-) schließt erfindungsgemäß beispielsweise lineare oder verzweigte Alkoxyreste bevorzugt mit bis zu 6 Kohlenstoffatomen ein, wie eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, eine n-Pentyloxygruppe, eine i-Pentyloxygruppe, eine sec-Pentyloxygruppe, eine t-Pentyloxygruppe, eine 2-Methylbutoxygrupe, eine n-Hexyloxygruppe, eine i-Hexyloxygruppe, eine t-Hexyloxygruppe, eine sec-Hexyloxygruppe, eine 2-Methylpentyloxygruppe, eine 3-Methylpentyloxygruppe, eine 1-Ethylbutyloxygruppe, eine 2-Ethylbutyloxygruppe, eine 1,1-Dimethylbutyloxygruppe, eine 2,2-Dimethylbutyloxygruppe, eine 3,3-Dimethylbutyloxygruppe, eine 1-Ethyl-1-methylpropyloxygruppe, usw. ein. Bevorzugt sind eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, usw. Die Alkoxygruppen können gegebenenfalls substituiert sein, wie beispielsweise mit den für Alkyl genannten möglichen Substituenten.

Bevorzugtes Alkoxy ist Methoxy, Ethoxy, n-Propoxy, i-Propoxy etc..

Gegebenenfalls substituierte Alkoxycarbonyl (RO-CO-)-Gruppen leiten sich aus den vorstehend definierten Alkylgruppen formal durch Hinzufügen eines -O-C(O)-Restes ab unter Bildung eines gegebenenfalls substituierten Alkyloxycarbonyl-Restes. Insoweit kann auf die Definition der vorstehend beschriebenen Alkylgruppen verwiesen werden. Alternativ leiten sich gegebenenfalls substituierte Alkoxycarbonyl (RO-CO-)-Gruppen aus den vorstehend genannten Alkoxygruppen entsprechend formal durch Hinzufügen einer Carbonylgruppe ab. Bevorzugte Alkoxycarbonyl-Gruppen sind Methoxycarbonyl, Ethoxycarbonyl, n-Propoxycarbonyl, n-Butoxycarbonyl, tert.-Butoxycarbonyl etc., welche sämtlich wie vorstehend definierte Alkylgruppen substituiert sein können.

Gegebenenfalls substituiertes Amino schließt erfindungsgemäß bevorzugt ein: Amino ($-NH_2$), gegebenenfalls substituiertes Mono- oder Dialkylamino (RHN-, $(R)_2N$-) wobei hinsichtlich der Definition von gegebenenfalls substituiertem Alkyl auf vorstehende Definition verwiesen werden kann. Ferner sind gegebenenfalls substituierte Mono- oder Diarylamino-Reste eingeschlossen oder gemischte gegebenenfalls substituierte Alkylarylamino-Reste, wobei hinsichtlich der Definition von gegebenenfalls substituiertem Alkyl oder Aryl auf vorstehende Definition verwiesen werden kann.

Bevorzugt ist mindestens ein Wasserstoffatom, bevorzugter beide Wasserstoffatome in Amino ($-NH_2$) insbesondere in der Definition von $R_3$ substituiert. Besonders bevorzugt ist gegebenenfalls substituiertes Amino in der Definition von $R_3$ gegebenenfalls substituiertes Mono- oder Dialkylamino (RHN-, $(R)_2N$-) insbesondere mit bis zu 12 Kohlenstoffatomen, wie zuvor erwähnt, oder es handelt sich um cyclisches Amino, bei dem das Stickstoffatom ein Ringatom eines cyclischen Amins mit bevorzugt bis zu 6 Ringkohlenstoffatomen ist, wie nachstehend erläutert.

Vorstehend genannte Alkyl- oder Arylreste in gegebenenfalls substituiertem Amino können beispielsweise 1 bis 3 Substituenten tragen, die beispielsweise ausgewählt werden aus der Gruppe der vorstehend für Alkyl genannten möglichen Substituenten, wie Hydroxy, gegebenenfalls substituiertem Aryl, insbesondere wie vorstehend definiert, gegebenenfalls substituiertem Heteroaryl, insbesondere wie vorstehend definiert, gegebenenfalls substituiertem Alkoxy insbesondere wie vorstehend definiert, gegebenenfalls substituiertem Alkoxycarbonyl, insbesondere wie vorstehend definiert, gegebenenfalls substituiertem Acyl, insbesondere wie nachstehend definiert, Halogen, insbesondere wie vorstehend definiert, gegebenenfalls substituiertem Amino, insbesondere wie nachstehend definiert, gegebenenfalls substituiertem Aminocarbonyl, insbesondere wie nachstehend definiert. Besonders bevorzugte Substituenten von Alkyl sind Hydroxy, Halogen, Alkoxy, Alkoxycarbonyl und Aminocarbonyl, jeweils wie zuvor erläutert.

Auch cyclisches Amino kann wie vorstehend erwähnt durch beispielsweise 1 bis 3 Substituenten substituiert sein, wobei hinsichtlich besagter Substituenten auf die zuvor für Alkyl an der Aminogruppe genannten verwiesen werden kann.

Derartige Amino-Gruppen schließen beispielsweise unsubstituertes Amino ($-NH_2$), Methylamino, Dimethyla-

mino, Ethylamino, Hydroxyethylamino, wie 2-Hydroxyethylamino, N-(Hydroxyethyl)-N-methyl-amino, Diethylamino, Phenylamino, Methylphenylamino etc. ein. Gegebenenfalls substituiertes Amino schließt weiterhin gegebenenfalls substituiertes cyclisches Amino ein, wie gegebenenfalls substituiertes 5 oder 6-gliedriges cyclisches Amino, dass gegebenenfalls weitere Heteroatome wie beispielsweise N, O, S, bevorzugt O enthalten kann. Beispiele von solchen cyclischen Aminogruppen schließen ein die vorstehend genannten stickstoffenthaltenden heterocyclischen Gruppen, die über Stickstoff gebunden sind, wie Piperidin-1-yl, 4-Hydroxy-piperidin-1-yl, 4-Alkoxypiperidin-1-yl, wie 4-Methoxypiperidin-1-yl, Piperazinyl, 4-Methyl-piperazinyl, 2-(Methoxycarbonyl)pyrrolidin-1-yl, Pyrrolidin-1-yl, Morpholin-4-yl, etc.

Bevorzugt ist gegebenenfalls substituiertes Amino insbesondere in der Definition von $R_3$: Amino ($-NH_2$), Mono- oder Dialkylamino, worin Alkyl substituiert sein kann, wie durch Hydroxy, wie insbesondere Dimethylamino, N-(Hydroxyethyl)-N-methyl-amino, sowie cyclisches, bevorzugt 5- oder 6-, noch bevorzugter 6-gliedriges cyclisches Amino wie Piperidino, Pyrrolidino und Morpholino, die jeweils substituiert sein können wie beispielsweise durch Alkoxy, wie 4-Methoxypiperidin-1-yl.

Gegebenenfalls substituiertes Acyl schließt im Rahmen der Erfindung aliphatisches und aromatisches Acyl ein, wobei aliphatisches Acyl insbesondere Formyl sowie gegebenenfalls substituiertes Alkylcarbonyl ist, wobei hinsichtlich der Definition des gegebenenfalls substituierten Alkyls auf die vorstehenden Erläuterungen verwiesen werden kann. Aromatisches Acyl schließt demzufolge gegebenenfalls substituiertes Arylcarbonyl ein, wobei hinsichtlich der Definition des gegebenenfalls substituierten Aryls auf die vorstehenden Erläuterungen verwiesen werden kann. Bevorzugte Acylgruppen stellen erfindungsgemäß beispielsweise dar: Formyl (-C(=O)H), Acetyl, Propionyl, Butanoyl, Pentanoyl, Hexanoyl und jeweils die Isomeren davon, Benzoyl. Substituenten von Acyl schließen die vorstehend für Alkyl und Aryl genannten Substituenten ein, so dass insoweit zu den diesbezüglichen Substituenten verwiesen werden kann.

Gegebenenfalls substituiertes Aminocarbonyl leitet sich im Rahmen der Erfindung formal von vorstehend definiertem gegebenenfalls substituierten Amino durch Hinzufügen eines Carbonylrestes ab ($(R)_2N-C(=O)-$), sodass insoweit auf vorstehende Definition von gegebenenfalls substituiertem Amino verwiesen werden kann. Beispiele schließen demnach ein Carbamoyl ($H_2NCO-$), gegebenenfalls substituiertes Mono- oder Dialkylaminocarbonyl ($RHNCO-$, $(R)_2NCO-$) wobei hinsichtlich der Definition von gegebenenfalls substituiertem Alkyl auf vorstehende Definition verwiesen werden kann. Ferner sind gegebenenfalls substituierte Mono- oder Diarylaminocarbonyl-Reste eingeschlossen oder gemischte gegebenenfalls substituierte Alkylarylaminocarbonyl-Reste, wobei hinsichtlich der Definition von gegebenenfalls substituiertem Alkyl oder Aryl auf vorstehende Definition verwiesen werden kann. Derartige Gruppen schließen beispielsweise Methylaminocarbonyl, Dimethylaminocarbonyl, Ethylaminocarbonyl, Diethylaminocarbonyl, Phenylaminocarbonyl, Methylphenylaminocarbonyl etc. ein.

Erfindungsgemäß bevorzugt sind Eisen(III)-Komplexverbindungen, enthaltend mindestens einen Liganden der Formel (I):

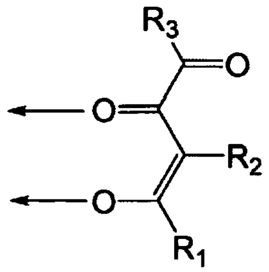

**(I)**

worin

die Pfeile jeweils eine koordinative Bindung zu einem oder verschiedenen Eisenatomen darstellen,

$R_1$ Alkyl ist, dass gegebenenfalls durch 1 bis 3 Substituenten substituiert sein kann, die aus der Gruppe ausgewählt sind, die besteht aus Hydroxy, Alkoxy, wie vorstehend definiert, wie insbesondere Methoxy, Ethoxy, Alkoxycarbonyl, wie vorstehend definiert, wie insbesondere Methoxycarbonyl, Ethoxycarbonyl, und Aminocarbonyl, wie vorstehend definiert, oder

$R_1$ Alkoxycarbonyl ist, dass gegebenenfalls durch 1 bis 3 Substituenten substituiert sein kann, die aus der Gruppe ausgewählt sind, die besteht aus Hydroxy und C1-C6-Alkoxy, wie insbesondere Methoxycarbonyl, Ethoxycarbonyl, Propoxycarbonyl etc. oder

$R_1$ Alkoxy ist, welches gegebenenfalls durch 1 bis 3 Substituenten substituiert sein kann, die aus der Gruppe ausgewählt sind, die besteht aus Hydroxy, Alkoxy und Halogen, jeweils wie vorstehend definiert,

$R_2$ ausgewählt wird aus der Gruppe, die besteht aus

- Wasserstoff,

- Alkyl, dass gegebenenfalls durch 1 bis 3 Substituenten substituiert sein kann, die aus der Gruppe ausgewählt sind, die besteht aus Hydroxy, Alkoxy, Halogen und Alkoxycarbonyl, jeweils wie vorstehend definiert,
- Halogen, wie Chlor, Fluor, besonders bevorzugt Fluor,

oder
$R_1$ und $R_2$ gemeinsam mit den Kohlenstoffatomen, an die sie gebunden sind, einen gegebenenfalls substituierten 5- oder 6-gliedrigen Ring bilden, wie einen Cyclopentanring oder einen Cyclohexanring, der gegebenenfalls auch ein oder mehrere, bevorzugt ein oder zwei Heteroatome aufweisen kann, und weitere, bevorzugt ein bis drei weitere Substituenten tragen kann, wie die für Alkyl genannten, und
$R_3$ Hydroxy ist, oder
$R_3$ Alkyl ist, dass gegebenenfalls durch 1 bis 3 Substituenten substituiert sein kann, die aus der Gruppe ausgewählt sind, die besteht aus Hydroxy, Alkoxy, Alkoxycarbonyl, jeweils wie vorstehend definiert, oder
$R_3$ Alkoxy ist, dass gegebenenfalls durch 1 bis 3 Substituenten substituiert sein kann, die aus der Gruppe ausgewählt sind, die besteht aus Hydroxy, Alkoxy, Alkoxycarbonyl, jeweils wie vorstehend definiert, oder
$R_3$ Amino ($-NH_2$) ist, dass gegebenenfalls durch bevorzugt 1 bis 3 Substituenten substituiert sein kann, die aus der Gruppe ausgewählt sind, die besteht aus Alkyl, Hydroxyalkyl, Alkoxy, Alkoxycarbonyl, jeweils wie vorstehend definiert, oder die gemeinsam einen gegebenenfalls substituierten 5- oder 6-gliedrigen Ring bilden (d.h. in diesem Fall ist $R_3$ ein cyclischer Aminorest, wie oben erläutert), wie einen Cyclopentanring oder einen Cyclohexanring, der gegebenenfalls auch ein oder mehrere, bevorzugt ein oder zwei Heteroatome aufweisen kann, und weitere, bevorzugt 1 bis drei weitere Substituenten tragen kann, wie die für Alkyl genannten,

oder pharmazeutisch verträgliche Salze davon.

Besonders bevorzugt sind insbesondere die Eisen(III)-Komplexverbindungen der allgemeinen Formel (II):

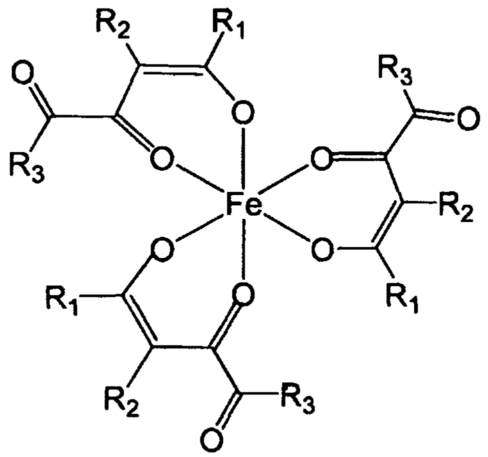

**(II)**

worin $R_1$, $R_2$, und $R_3$ jeweils wie oben oder bevorzugt wie nachstehend definiert sind.

In einer bevorzugten Ausführungsform der Erfindung wird $R_1$ ausgewählt aus der Gruppe, die besteht aus:

- $C_{1-6}$-Alkyl, bevorzugt wie vorstehend erläutert, gegebenenfalls substituiert mit $C_{1-4}$-Alkoxy, wie vorstehend erläutert, oder gegebenenfalls substituiert mit Alkoxycarbonyl, wie vorstehend erläutert,
- $C_{3-6}$-Cycloalkyl, bevorzugt wie vorstehend erläutert,
- $C_{3-6}$-Cycloalkyl-$C_{1-4}$-alkyl, bevorzugt wie vorstehend erläutert,
- $C_{1-4}$-Alkoxy, bevorzugt wie vorstehend erläutert.
- Hydroxy-$C_{1-4}$-alkyl, bevorzugt wie vorstehend erläutert, und
- Halogen-$C_{1-4}$-alkyl, bevorzugt wie vorstehend erläutert, oder
- $C_{1-4}$-Alkoxycarbonyl, bevorzugt wie vorstehend erläutert.

Besonders bevorzugt ist $R_1$ $C_{1-6}$-Alkyl, bevorzugt wie vorstehend erläutert, insbesondere Methyl, Ethyl, Propyl, insbesondere n-Propyl und i-Propyl, Butyl, wie insbesondere tert.-Butyl, . Am meisten bevorzugt ist $R_1$ Methyl, Ethyl, i-Propyl (Isopropyl) und tert.-Butyl, welche gegebenenfalls durch $C_{1-6}$-Alkoxy, wie Methoxy substituiert sein kann, oder
$R_1$ ist $C_{1-4}$-Alkoxycarbonyl, wie insbesondere Ethoxycarbonyl oder Methoxycarbonyl, oder
$R_1$ ist $C_{1-4}$-Alkoxy, bevorzugt wie vorstehend erläutert, insbesondere Methoxy und Ethoxy.
In einer bevorzugten Ausführungsform der Erfindung wird $R_2$ ausgewählt aus der Gruppe, die besteht aus:

- Wasserstoff,
- Halogen, bevorzugt wie vorstehend erläutert,
- $C_{1-6}$-Alkyl, bevorzugt wie vorstehend erläutert.

Besonders bevorzugt ist $R_2$ Wasserstoff und Halogen jeweils bevorzugt wie vorstehend erläutert, bevorzugter ist $R_2$ Wasserstoff oder Fluor, am meisten bevorzugt Wasserstoff, oder $R_2$ bildet mit $R_1$ eine ringförmige Struktur wie nachstehend beschrieben.

In einer bevorzugten Ausführungsform der Erfindung können $R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere (wie insbesondere 2) Heteroatome aufweisen kann. Dann liegen schematisch 2,4-Dioxocarbonyl-Liganden der folgenden Formel vor:

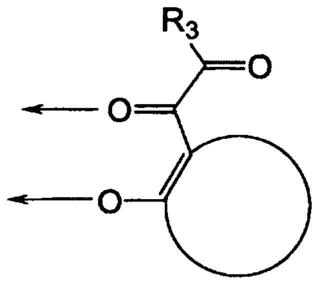

worin $R_3$ wie vorstehend oder nachstehend beschrieben ist. Diese Ausführungsform ist allerdings weniger bevorzugt.

In dieser Ausführungsform stellen $R_1$ und $R_2$ bevorzugt zusammen eine Propylen($-CH_2-CH_2-CH_2-$)- oder eine Butylen ($-CH_2-CH_2-CH_2-CH_2-$)-Gruppe dar, in denen jeweils eine Methylengruppe ($-CH_2-$) durch -O-, -NH-, oder $-NR_4$- ersetzt sein kann, worin $R_4$ gegebenenfalls substituiertes Alkyl ist, und worin die von $R_1$ und $R_2$ gebildeten Gruppen weiterhin jeweils durch einen bis drei Substituenten, ausgewählt aus der Gruppe, die besteht aus Hydroxy, $C_{1-4}$-Alkoxy, Amino und Mono- oder Di-($C_{1-4}$-alkyl)amino, substituiert sein können. Beispielhafte bevorzugte Liganden dieses Typs sind die folgenden:

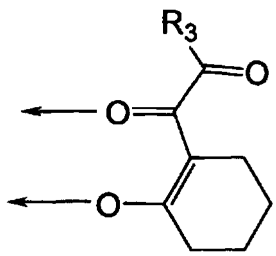

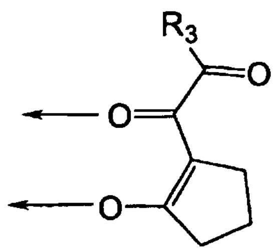

worin $R_3$ jeweils wie oben definiert ist.

In einer bevorzugten Ausführungsform der Erfindung wird $R_3$ ausgewählt aus der Gruppe, die besteht aus:

- $C_{1-6}$-Alkyl, bevorzugt wie vorstehend erläutert, gegebenenfalls substituiert mit $C_{1-4}$-Alkoxy, wie vorstehend erläutert, oder
- Hydroxy, oder
- $C_{1-4}$-Alkoxy, bevorzugt wie vorstehend erläutert, oder
- Amino ($NH_2$), wie vorstehend erläutert, gegebenenfalls substituiert mit $C_{1-6}$-Alkyl, bevorzugt wie vorstehend erläutert, oder cyclisches, bevorzugt 5-oder 6-gliedriges, gegebenenfalls substituiertes Amino, das gegebenenfalls weitere Heteroatome, wie ein oder zwei Heteroatome, bevorzugt N oder O, neben dem Stickstoffatom über welchen $R_3$ gebunden ist, aufweisen kann und am Ring substituiert sein kann, wie durch Hydroxy oder Alkoxy. Beispielhafte Reste für cyclische Amino-Reste $R_3$ sind die folgenden:

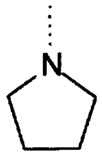 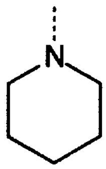 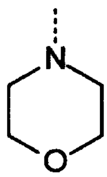 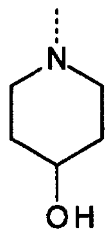 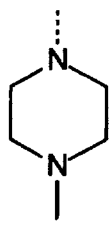 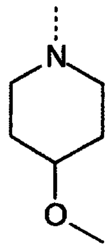

Hierbei ist --- die Bindungsstelle, über die der Rest $R_3$ gebunden ist.

Besonders bevorzugt ist $R_3$ Hydroxy, Alkoxy und gegebenenfalls substituiertes Amino, am meisten bevorzugt Hydroxy, Methoxy, Ethoxy, Amino (-NH2), Dimethylamino, N-(Hydroxyethyl)-N-methyl-amino:

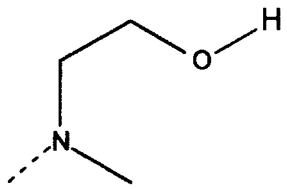

,

Piperidin-1-yl, 4-Methoxypiperidin-1-yl, Pyrrolidin-1-yl und Morpholin-4-yl.

Es ist für den Fachmann klar, dass die erfindungsgemäßen Liganden

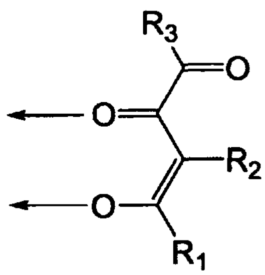

aus den entsprechenden 2,4-Dioxo-1-carbonylverbindungen hervorgehen:

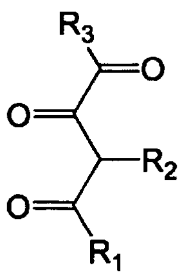

,

in denen bekanntermaßen eine Keto-Enol-Tautomerie vorliegt:

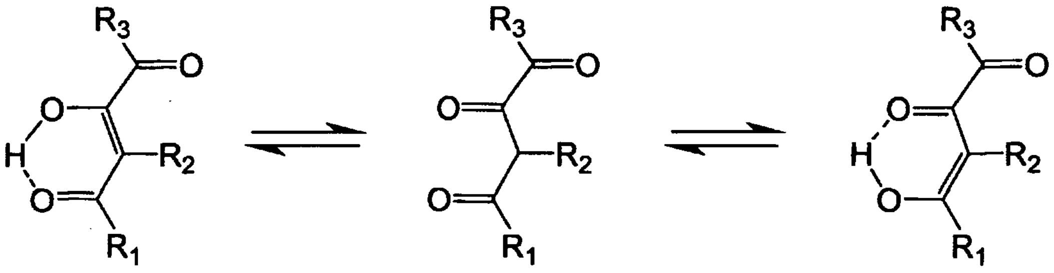

A B C .

Die mesomeren Grenzformen A und C sind analytisch so gut wie nicht unterscheidbar. Ausnahmen hierbei sind nur im Fall wenn $R_1$ und $R_3$ sehr unterschiedlich sind. Im Rahmen der vorliegenden Erfindung sind in jedem Fall sämtliche Formen eingeschlossen, wobei jedoch im Rahmen der vorliegenden Erfindung bei den Liganden im Allgemeinen nur die Ketoform gezeichnet ist.

Der Ligand geht formal durch Abspalten eines Protons aus den entsprechenden 2,4-Dioxocarbonylverbindungen hervor:

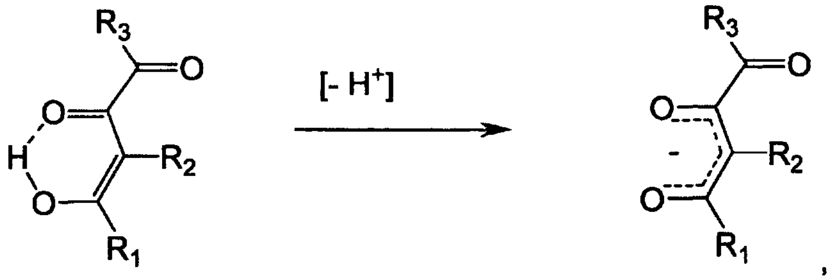


,

trägt also formal eine einfach negative Ladung. Auch bei den EisenKomplexverbindungen ist im Rahmen der vorliegenden Erfindung stets lediglich eine der beiden lokalisierten Grenzformeln gezeigt:

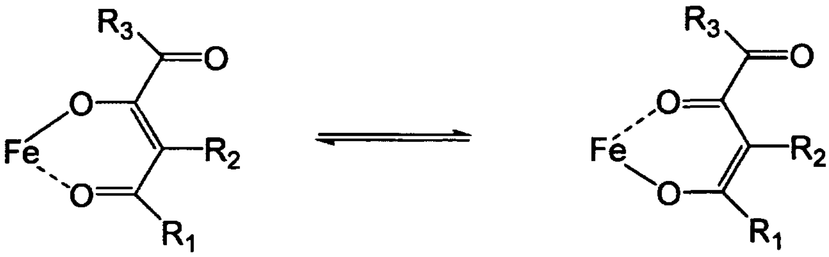

A C .

Weitere bevorzugte Ausführungsformen der Erfindung schließen ein:

$R_1$ wird ausgewählt aus der Gruppe, die besteht aus:

- Methyl
- i-Propyl (Isopropyl)
- t-Butyl (tertiär-Butyl)
- Methoxy
- Ethoxy
- Methoxycarbonyl und
- Ethoxycarbonyl, und

$R_2$ wird ausgewählt aus der Gruppe, die besteht aus:

- Wasserstoff
- Methyl, und
- Fluor oder

$R_1$ und $R_2$ bilden zusammen eine Propylen ($-CH_2-CH_2-CH_2-$) oder Butylen ($-CH_2-CH_2-CH_2-CH_2-$) Gruppe und $R_3$ wird ausgewählt aus der Gruppe, die besteht aus:

- Hydroxy,
- Methoxy
- Ethoxy
- Amino
- Dimethylamino
- N-(Hydroxyethyl)-N-methyl-amino
- 3-Propylamino (n-Propylamino)
- 2-Ethylamino (Ethylamino)
- Morpholino (Morpholin-4-yl)
- Piperidino (Piperidin-1-yl)
- Pyrrolidino (Pyrrolidin-1-yl)
- 4-Hydroxypiperidino (4-Hydroxypiperidin-1-yl)
- 4-Methoxypiperidino (4-Methoxypiperidin-1-yl).

(In der vorliegenden Erfindung bedeuten die Ziffern 1-6 in "1-6C" oder "C1-6", bzw. "1-4" in "1-4C" oder "C1-4 "etc. jeweils die Anzahl der Kohlenstoffatome der sich daran anschließenden Kohlenwasserstoffreste-Bezeichnungen).

Besonders bevorzugt werden die vorstehend genannten Substituentengruppen wie folgt definiert:

**1-6C-Alkyl** beinhaltet bevorzugt geradkettige oder verzweigte Alkylgruppen mit 1 bis 6 Kohlenstoffatomen. Beispiele hierfür können Methyl, Ethyl, n-Propyl, iso-Propyl, n-Butyl, iso-Butyl, sec-Butyl, tert-Butyl n-Pentyl, iso-Pentyl, neo-Pentyl, n-Hexyl, isoHexyl und neo-Hexyl sein.

**3-6C-Cycloalkyl** beinhaltet bevorzugt Cycloalkyl mit 1 bis 6 Kohlenstoffatomen, wie Cyclopropyl, Cyclobutyl, Cyclopentyl, Cyclohexyl.

**3-6C-Cycloalkyl-1-4C-alkyl** beinhaltet bevorzugt eine oben beschriebene 1-6C-Alkyl Gruppe, substituiert mit einer oben beschriebenen 3-6C-Cycloalkyl Gruppe. Beispiele hierfür können eine Cyclopropylmethyl, Cyclopentylmethyl und Cyclohexylmethyl Gruppe sein.

**1-3C-alkoxy-carbonyl-1-6C-alkyl,** beinhaltet bevorzugt eine oben beschriebene 1-6C-Alkyl Gruppe, die mit einer Carbonylgruppe, die mit einer 1-3C Alkoxy Gruppe als Carbonsäureester vorliegt, verknüpft ist. Beispiele hierfür können ein Methoxycarbonylmethyl, Ethoxycarbonylmethyl, Methoxycarbonylethyl, Ethoxycarbonylethyl und Isopropoxycarbonylmethyl.

**1-4C-Alkoxy** beinhaltet bevorzugt eine 1-4C-Alkoxy Gruppe, bei der ein Sauerstoffatom mit einer geraden oder verzweigten Alkylkette mit 1-4 Kohlenstoffatome verbunden ist. Beispiele dieser Gruppe können Methoxy, Ethoxy, Propoxy und Isobutoxy sein.

**1-4C-Alkoxy-1-4C-alkyl** beinhaltet bevorzugt eine oben beschriebene 1-4C-Alkoxy Gruppe, die mit einer oben beschriebenen 1-4C-alkyl Gruppe verknüpft ist. Beispiele dieser Gruppe können Methoxyethyl, Ethoxypropyl, Methoxypropyl, Isobutoxymethyl sein.

**Hydroxy-1-4C-alkyl** beinhaltet eine oben beschriebene 1-4C-Alkyl Gruppe, die mit einer Hydroxygruppe substituiert ist. Beispiele hier können Hydroxyethyl, Hydroxybutyl und Hydroxyisopropyl sein.

**Fluor-1-4C-alkyl** beinhaltet eine oben beschriebene 1-4C-Alkyl Gruppe, die mit eins bis drei Fluoratomen substituiert ist. Beispiele hier können Trifluormethyl und Trifluorethyl sein.

**Halogen** bedeutet F, Cl, Br, I.

Besonders bevorzugte Eisen(III)-Komplex-Verbindungen der allgemeinen Formel (II) sind in den Beispielen

beschrieben.

Die Beschreibung betrifft weiterhin ein Verfahren zur Herstellung der erfindungsgemäßen Eisen(II)-Komplexverbindungen, welches die Umsetzung eines 2,4-Dioxocarbonylverbindung mit einem Eisen(III)-salz umfasst.

2,4-Dioxo-1-carbonyl-Verbindungen schließen insbesondere diejenigen der Formel (III) ein:

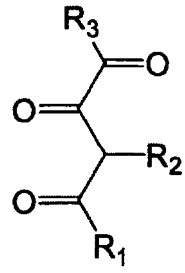

(III)

worin $R_1$ bis $R_3$ jeweils wie oben definiert sind, wobei auf die tautomeren Grenzstrukturen hingewiesen wurde.

Beispiele geeigneter Eisen(III)-salze schließen ein: Eisen(III)-chlorid, Eisen(III)-acetat, Eisen(III)-sulfat, Eisen(III)-nitrat, Eisen(III)-ethoxid, und Eisen(III)-acetylacetonat, worunter Eisen(III)-chlorid bevorzugt ist.

Ein bevorzugtes Verfahren ist im folgenden Schema gezeigt:

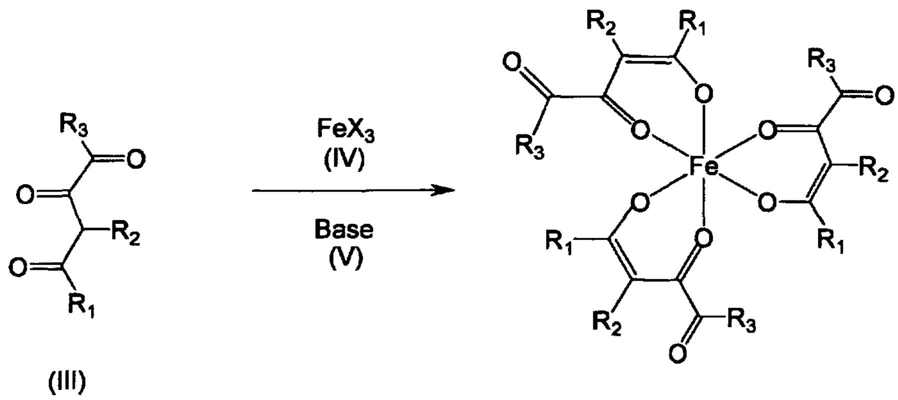


(III)

(II)

,

worin $R_1$ bis $R_3$ jeweils wie oben definiert sind, X ein Anion wie Halogenid, wie Chlorid, ein Carboxylat, wie Acetat, Sulfat, Alkoxy, wie Ethoxy, Nitrat und Acetylacetonat ist und die optional verwendete Base (V) eine übliche organische oder anorganische Base ist.

Beim erfindungsgemäßen Verfahren werden bevorzugt 3-5 eq Ligand (III) unter Verwendung von geeigneten Eisen(111)-salzen (IV) (besonders eignen sich hier Fe(III)-chlorid, Fe(III)-acetat, Fe(III)-ethoxid, Fe(III)-sulfat und Fe(III)-acetylacetonat) zu den entsprechenden Komplexen der allgemeinen Formel (II) unter Standardbedingungen umgesetzt. Hierbei wird die Synthese unter den für die Komplexbildung optimalen pH-Bedingungen durchgeführt. Der optimale pH-Wert wird gegebenenfalls durch Zugabe von Base (V), besonders eignet sich hier die Verwendung von Triethylamin, Natrium-Carbonat, Natrium-Hydrogencarbonat, Natrium-Methanolat, Natrium-Ethanolat, Kaliumcarbonat, Kalium-Hydrogencarbonat oder Kalium-Methanolat, eingestellt.

Die für die Darstellung der Komplexe benötigten Liganden (III) sind entweder käuflich erhältlich oder wurden nach folgender Synthesemethode dargestellt.

Hierzu wurden unterschiedliche Synthesewege beschritten. Für die Liganden der allgemeinen Formel $R_1$ = Methyl, $R_2$ =H, $R_3$ = $NH_2$ oder OH wurde der käufliche Ester Ethyl 2,4-dioxopentanoat im Fall von $R_1$ = Methyl, $R_2$ =H, $R_3$ = $NH_2$ in Form seines Calciumsalzes mit Ammoniak zum Liganden der allgemeinen Formel (III) umgesetzt (A. Ichiba et al, *Journal of the Scientific Research Institute, Tokyo,* **1948,** *23*, 23-29).

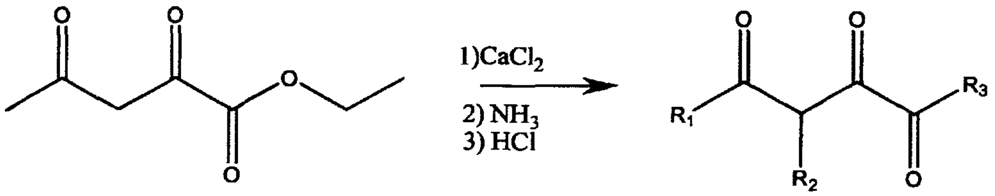


Im Fall von $R_1$ = Methyl, $R_2$ =H, $R_3$ =$N(CH_3)CH_2CH_2OH$ wurde Ethyl 2,4-dioxopentanoat in Form seines Kupferkomplexes mit 2-Methylaminoethanol zum Liganden der allgemeinen Formel (III) umgesetzt.

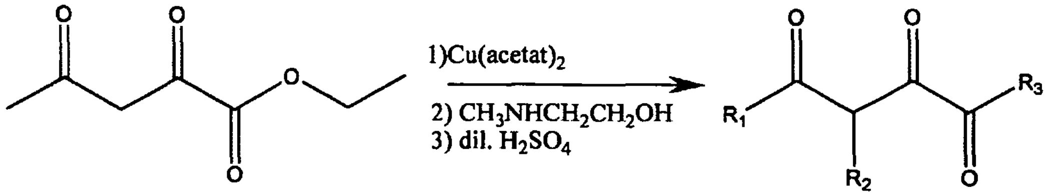


Im Fall von $R_1$ = Methyl, $R_2$ =H, $R_3$ =OH wurde Ethyl 2,4-dioxopentanoat durch basische Hydrolyse zum Liganden der allgemeinen Formel (III) umgesetzt.

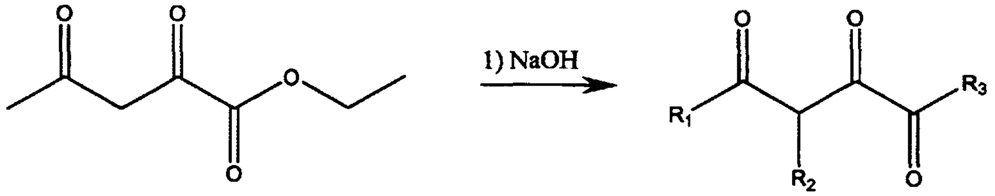


Für die anderen Liganden der allgemeinen Formel (III) wurden basische Kondensationsreaktionen verwendet (J. Wang et al, Can. J. Chem., 2009, 87, 328-334).

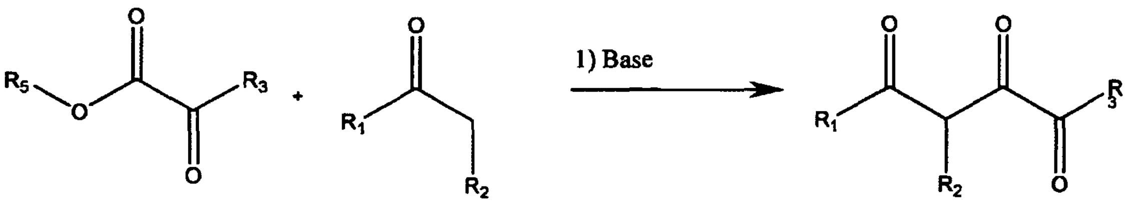


worin $R_1$ bis $R_3$ jeweils wie oben definiert sind und $R_5$ ein gegebenenfalls substituierten Alkylrest, bevorzugt jedoch einen Methyl- oder Ethylrest darstellt. Als Basen kommen verschiedene Kondensationsbasen wie Natrium-ethylat, Kalium-tert-butylat, Natrium, Natriumhydrid oder Buthyllithium in Frage, wobei Kalium-tert-butylat bevorzugt ist.

Liganden, welche in $R_3$ eine freie Hydroxy-Gruppe -OH enthalten, können bei der Umsetzung mit den Carbonylgruppen des Grundgerüsts Halbacetale ausbilden. In diesen Fällen ändert sich die allgemeine Formel (III) des nicht an Eisen gebundenen Liganden zur allgemeinen Formel (VI):

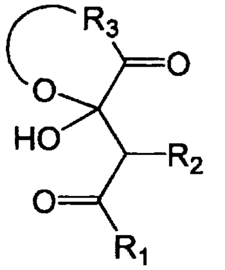

(VI).

Für den Fachmann ist klar, dass Halbacetale der Formel (VI) in Lösung im Gleichgewicht mit der offenkettigen Form der allgemeinen Formel (III) stehen und bei Koordination an Eisen stets als Liganden der allgemeine Formel (I) vorliegen.

Pharmazeutisch annehmbare Salze der erfindungsgemäßen Verbindungen, in denen der Eisen(III)-Komplex formal eine positive Ladung trägt, schließen beispielsweise Salze mit geeigneten Anionen ein, wie Carboxylate, Sulfonate, Sulfate, Chloride, Bromide, Iodide, Phosphate, Tartrate, Methansulfonate, Hydroxyethansulfonate, Glycinate, Maleate, Propionate, Fumarate, Toluolsulfonate, Benzolsulfonate, Trifluoracetate, Naphthalindisulfonate-1,5, Salicylate, Benzoate, Lactate, Salze der Äpfelsäure, Salze der 3-Hydroxy-2-naphthoesäure-2, Citrate und Acetate ein.

Pharmazeutisch annehmbare Salze der erfindungsgemäßen Verbindungen, in denen der Eisen(III)-Komplex formal eine negative Ladung trägt, schließen beispielsweise Salze mit geeigneten pharmazeutisch annehmbaren Basen ein, wie z.B. Salze mit Alkali- oder Erdalkalihydroxiden, wie NaOH, KOH, $Ca(OH)_2$, $Mg(OH)_2$ etc., Aminverbindungen, wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3), 2-Amino-2-hydroxylmethyl-propandiol-(1,3) (TRIS) etc..

Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.

Bevorzugt stellen die erfindungsgemäßen Verbindungen neutrale Komplex-Verbindungen dar.

**Vorteilhafte pharmakologische Effekte:**

Die Erfinder fanden überraschend, dass die Eisen(III)-2,4-dioxocarbonyl-Komplexverbindungen, die Gegenstand der vorliegenden Erfindung sind, und die durch die allgemeine Strukturformel (II) dargestellt werden, stabile bioverfügbare Eisenkomplexe sind und zur Verwendung als Arzneimittel zur Behandlung und Prophylaxe von Eisenmangelerscheinungen und Eisenmangelanämien sowie der damit einhergehenden Symptome geeignet sind.

Die erfindungsgemäßen Verbindungen enthaltende Arzneimittel eignen sich zum Einsatz in der Human- und der Veterinärmedizin.

Die erfindungsgemäßen Verbindungen eignen sich somit auch zur Herstellung eines Medikamentes zur Behandlung von Patienten, die unter Symptomen einer Eisenmangelanämie wie zum Beispiel: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit oder depressiven Verstimmungen leiden.

Die erfindungsgemäßen Eisen(III)-Komplexverbindungen eignen sich weiterhin zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3 -5 (CDK 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases) sowie Eisenmangelanämien bei Malaria.

Die Verabreichung kann über einen Zeitraum von mehreren Monaten bis zur Verbesserung des Eisenstatus, reflektiert beispielsweise durch den Hämoglobin-Wert, die Transferrin-Sättigung und den Serum Ferritin-Wert der Patienten, oder zur gewünschten Verbesserung einer durch Eisenmangelanämie hervorgerufenen Beeinträchtigung des Gesundheitszustandes erfolgen.

Das erfindungsgemäße Präparat kann von Kindern, Jugendlichen und Erwachsenen eingenommen werden.

Die erfindungsgemäßen Verbindungen angewendeten Verbindungen können dabei sowohl oral als auch parenteral verabreicht werden. Bevorzugt ist die orale Verabreichung.

Die erfindungsgemäßen Verbindungen sowie die vorgenannten Kombinationen der erfindungsgemäßen Verbindungen mit weiteren Wirkstoffen oder Arzneimitteln können somit insbesondere zur Herstellung von Medikamenten

zur Behandlung der Eisenmangelanämie verwendet werden, wie Eisenmangelanämien bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäße Anwendung führt zu einer Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder zu einer Verbesserung der Neutrophilen-Niveaus, der Antikörperniveaus und/oder der Lymphozytenfunktion einhergeht.

Die vorliegende Erfindung betrifft weiterhin pharmazeutische Zusammensetzungen, enthaltend eine oder mehrere der erfindungsgemäßen Verbindungen, insbesondere nach Formel (II), sowie gegebenenfalls eine oder mehrere weitere pharmazeutisch wirksame Verbindungen sowie gegebenenfalls einen oder mehrere pharmakologisch verträgliche Träger und/oder Hilfsstoffe und/oder Lösungsmittel. Die genannten pharmazeutische Zusammensetzungen enthalten beispielsweise bis 99 Gew.-% oder bis zu 90 Gew.-% oder bis zu 80 Gew.-% oder bis zu 70 Gew.-% der erfindungsgemäßen Verbindungen, wobei der Rest jeweils durch pharmakologisch verträgliche Träger und/oder Hilfsstoffe und/oder Lösungsmittel gebildet wird.

Dabei handelt es sich um übliche pharmazeutische Träger, Hilfsstoffe oder Lösungsmittel. Genannte pharmazeutische Zusammensetzungen sind beispielsweise geeignet zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Granulaten, Suppositorien, Emulsionen, Dispersionen, Mikrokapseln, Mikroformulierungen, Nanoformulierungen, liposomalen Formulierungen, Kapseln, magensaftresistenten Kapseln, Pulvern, Inhalationspulvern, mikrokristallinen Formulierungen, Inhalationssprays, Puder, Tropfen, Nasentropfen, Nasensprays, Aerosolen, Ampullen, Lösungen, Säften, Suspensionen, Infusionslösungen oder Injektionslösungen etc. vor.

In einer bevorzugten Ausführungsform der Erfindung werden die EisenKomplexverbindungen in Form einer Tablette oder Kapsel verabreicht. Diese könnenbeispielsweise als säureresistente Formen oder mit pH-abhängigen Beschichtungen vorliegen.

Bevorzugt werden die erfindungsgemäßen Verbindungen sowie pharmazeutische Zusammensetzungen, enthaltend solche Verbindungen oral appliziert, obwohl auch andere Formen wie parenteral, insbesondere intravenös möglich sind.

Dazu liegen die erfindungsgemäßen Verbindungen bevorzugt in pharmazeutischen Zusammensetzungen in Form von Pillen, Tabletten, magensaftresistenten Tabletten, Filmtabletten, Schichttabletten, Retardformulierungen zur oralen Verabreichung, Depotformulierungen, Dragees, Granulaten, Emulsionen, Dispersionen, Mikrokapseln, Mikroformulierungen, Nanoformulierungen, liposomalen Formulierungen, Kapseln, magensaftresistenten Kapseln, Pulvern, mikrokristallinen Formulierungen, Puder, Tropfen, Ampullen, Lösungen, Suspensionen, Infusionslösungen oder Injektionslösungen vor.

Die erfindungsgemäßen Verbindungen können in pharmazeutischen Zusammensetzungen verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmittel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssige Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heisst diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Der Wirkstoff kann beispielsweise mit einer Einheitsdosis von 0,001 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

## EXPERIMENTELLER TEIL

Die Ligandbezeichnungen wurden nach IUPAC-Nomenklatur mit dem Programm ACD/Name, Version 12.01 von Advanced Chemistry Development Inc. erstellt.

**Ausgangsverbindungen:**

### A. Ethyl 2,4-dioxopentanoat

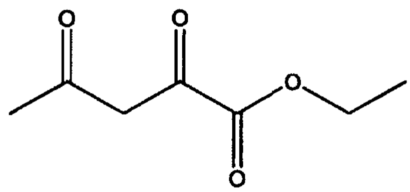

Kommerziell erhältlich: Sigma-Aldrich 232564

### B. Methyl 2,4-dioxopentanoat

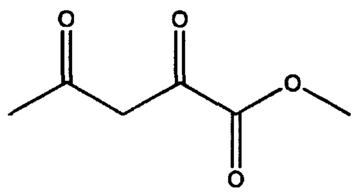

Kommerziell erhältlich: Sigma-Aldrich 699675

### C. 2,4-Dioxopentanamid

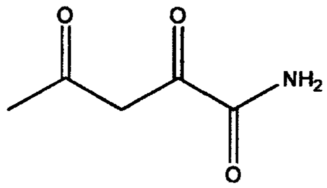

100g Calcium Acetat Monohydrat wurden in 250ml Wasser gelöst und im Eisbad gut abgekühlt. 185g Ethyl 2,4-dioxovalerat wurden mit einem Tropftrichter innerhalb 30 min zugegeben. Man rührt unter Eisbadkühlung mit mechanischem Rührer für 2h. Der entstandene Niederschlag wurde abfiltriert und zur Entfernung der Essigsäure gut mit 100ml Eiswasser nachgewaschen. Der Niederschlag wurde im Trockenschrank bei 25°C zur Gewichtskonstanz getrocknet, sorgfältig im Mörser pulverisiert und nochmals über Nacht bei 25°C im Trockenschrank nachgetrocknet. Ausbeute: 178g des Calciumsalzes von Ethyl-2,4-dioxovalerat in Form eines weissen Pulvers.
25g des Calciumsalzes von Ethyl-2,4-dioxovalerat wurden bei Raumtemperatur unter Feuchtigkeitsausschluss mit 50ml ammoniakalischem Methanol (7N) versetzt und 1 h gerührt. Anschliessend liess man ungerührt für 24 Stunden stehen. Der Kolbeninhalt verfestigte sich in dieser Zeit komplett. Man schlämmte zur Aufarbeitung mit 100ml Methanol auf und filtriert den weissen Feststoff ab. Man trocknete für 3h bei 40°C im Feinvakuum. Als Ausbeute wurden 15g des Calciumsalzes von 2,4-Dioxopentanamid als weisser Feststoff erhalten.
Zur Freisetzung von 2,4-Dioxopentanamid wurden 20g des getrockneten Calciumkomplexes zu einem feinen Pulver gemörsert in einem Kolben vorgelegt und in einer Kältemischung gekühlt. Unter Rühren wurden dann 40g vorgekühlte 20%ige Salzsäure zügig zugetropft. Man rührte für 30min bei -10°C Innentemperatur bis der pH-Wert der Suspension konstant war. Die eiskalte Suspension wurde schnell filtriert, mit wenig Eiswasser gewaschen und im Feinvakuum bei Raumtemperatur getrocknet. Das Rohprodukt wurde aus Ethanol umkristallisiert. Die Ausbeute an 2,4-Dioxopentanamid betrug 7g in Form eines beigen Pulvers mit Schmelzpunkt bei 130°-132°C.
IR (in Substanz, $cm^{-1}$): 3417, 3290, 3195, 3119, 2773, 1677, 1579, 1378, 1220, 1108, 1022, 991, 933, 836, 798, 673.
Enolform
$^1$H-NMR (DMSO-d6, 400 MHz): $\delta$ [ppm] = 8.05 (1 H), 7.86 (1H), 6.32 (1 H), 2.19 (3H).
Ketoform
$^1$H-NMR (DMSO-d6, 400 MHz): $\delta$ [ppm] = 7.97 (1H), 7.71 (1H), 3.91 (2H), 2.19 (3H).

**D. 2,4-Dioxopentansäure**

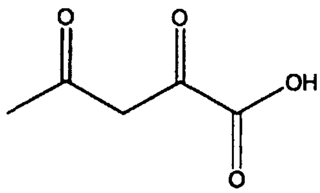

Es wurden 50.00 g (316 mmol) 2,4-Dioxopentansäuremethylester mit 25 ml Aceton versetzt und auf 0 °C abgekühlt. Anschliessend wurden bei -3 bis +5 °C 126.5 ml 5 M Natronlauge zugegeben und für 4 h bei 0 °C nachgerührt. Zu der Lösung wurden 106.0 ml 3 M Schwefelsäure so zugetropft, dass die Temperatur im Bereich von 0 bis 5 °C gehalten wurde. Die Mischung wurde filtriert und das Filtrat in einen Kutscher-Steudel-Extraktor gegeben und 6½ h mit Diethylether extrahiert. Die org. Phase wurde abgetrennt, über Natriumsulfat getrocknet und bis zur Trockene eingeengt. Der Rückstand wurde aus 15 ml n-Hexan / Ethylacetat (1:1) umkristallisiert und nach 16 stündigem aufbewahren bei 4 °C wurde die Säure abfiltriert. Das erhaltene Rohprodukt wurde in eine Sublimationsapparatur gegeben und für 10½ h bei 50 bis 60 °C und 6 bis 10 mbar sublimiert. Hierbei wurden 7.88 g 2,4-Dioxopentansäure in Form eines weissen Pulvers erhalten.
IR (in Substanz, $cm^{-1}$): 3186, 3114, 3033, 2935, 2577, 1756, 1736, 1586, 1380, 1296, 1237, 1194, 1129, 997, 901, 832, 792, 713, 561.
Enolform
$^1$H-NMR (DMSO-d6, 400 MHz): $\delta$ [ppm] = 13.3 (1 H), 6.27 (2 H), 2.24 (3H). Ketoform
$^1$H-NMR (DMSO-d6, 400 MHz): $\delta$ [ppm] = 13.6 (1 H), 3.96 (2 H), 2.18 (3 H).

**E. Ethyl oxo(2-oxocyclopentyl)acetat**

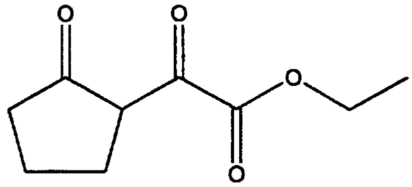

Kommerziell erhältlich: Akos GmbH MSC-0387

**F. Ethyl oxo(2-oxocyclohexyl)acetat**

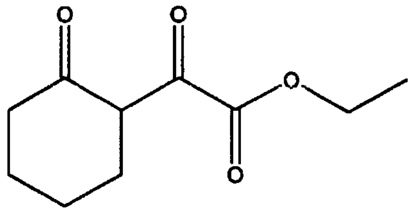

Kommerziell erhältlich: Akos GmbH MSC-0385

**G. 5,5-Dimethyl-1-(piperidin-1-yl)hexan-1,2,4-trion**

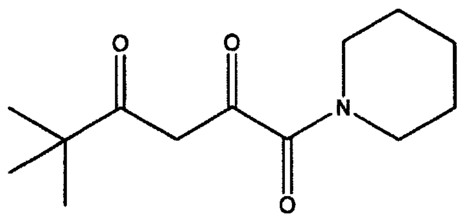

14.0 g (164 mmol) Piperidin und 16.6 g (164 mmol) Triethylamin wurden in 30 ml Dichlormethan auf 0°C abgekühlt. Man gab 20.1 g (164 mmol) Methyl chloro-oxoacetat tropfenweise zu. Anschliessend liess man für 16 Stunden bei Raumtemperatur rühren. Die Reaktionslösung wurde zuerst mit 1 N Salzsäure und danach mit gesättigter Natriumhydrogencarbonatlösung gewaschen, über Natriumsulfat getrocknet und am Rotationsverdampfer zur Trockene eingeengt. Von dem zurückgebliebenen Öl wurden 5g zusammen mit 2.93 g (29.3mmol) 3,3-Dimethyl-2-butanon in 5ml trockenem THF vorgelegt und unter Kühlung 6.89g (61.4 mmol) Kalium-tert-butanolat portionsweise in 40 Minuten zugegeben. Die Farbe der Reaktionsmischung wechselte von dunkelgelb nach grüngelb. Anschliessend wurde noch 3 Stunden bei Raumtemperatur gerührt. Man gab 6.32 g (105 mmol) Eisessig zu und filtrierte die Suspension. Das Filtrat wurde mit gesättigter NaCl-Lösung und 5%iger Natriumhydrogencarbonat-Lösung gewaschen. Beide Waschlösungen wurden abschliessend mit Dichlormethan extrahiert und dieses mit dem Filtrat vereinigt. Man trocknete über Natriumsulfat und engte am Rotationsverdampfer zur Trockene ein. Das Rohprodukt wurde säulenchromatographisch (Kieselgel, Ethylacetat/Ethanol = 13/1) gereinigt, die Ausbeute betrug 2.6g in Form eines gelben Öls.
LC/MS : m/z [Da] = 240.5 $[M+H]^+$, 262.3 $[M+Na]^+$.
$^1$H-NMR (CDCl3, 400 MHz): $\delta$ [ppm] = 5.88 (1H), 3.6-3.4 (4H), 1.7-1.5 (6H), 1.17 (9H).

**H. N,N,5,5-tetramethyl-2,4-dioxohexanamid**

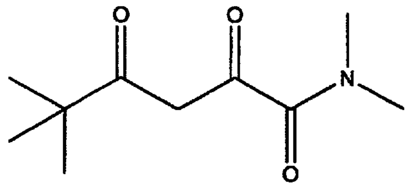

15 g (103 mmol) Ethyl N,N-dimethyloxamat wurden zusammen mit 10.4 g (103 mmol) 3,3-Dimethyl-2-butanon in 40ml trockenem THF vorgelegt. 24.6 g (217 mmol) Kalium-tert-butanolat wurden in 200 ml trockenem THF suspendiert und in 40 Minuten zur Reaktionsmischung getropft. Die Reaktionsmischung färbte sich dabei orange. Anschliessend wurde noch 3 Stunden bei Raumtemperatur gerührt. Man gab 22.3 g (371 mmol) Eisessig zu und filtrierte die Suspension. Der abfiltrierte Feststoff wurde noch mit 300 ml Dichlormethan nachgewaschen und mit dem Filtrat vereint. Die organische Phase wurde mit 100 ml 5%iger Natriumhydrogencarbonat-Lösung und 100 ml gesättigter NaCl-Lösung gewaschen, über Natriumsulfat getrocknet und am Rotationsverdampfer zur Trockene eingeengt. Das Rohprodukt wurde säulenchromatographisch (Kieselgel, Ethylacetat/Heptan = 1/1) gereinigt, die Ausbeute betrug 10.2 g in Form eines gelben Öls.
LC/MS : m/z [Da] = 200.3 $[M+H]^+$, 222.3 $[M+Na]^+$.
Enolform
$^1$H-NMR (DMSO-d6, 400 MHz): $\delta$ [ppm] = 5.92 (1 H), 3.03 (3H), 2.94 (3H), 1.13 (9H).
Ketoform
$^1$H-NMR (CDCl3, 400 MHz): $\delta$ [ppm] = 3.97 (2H), 3.06 (3H), 2.92 (3H), 1.11 (9H).

**I. N,N,5-trimethyl-2,4-dioxohexanamid**

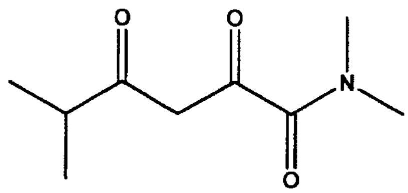

19.6 g (135 mmol) Ethyl N,N-dimethyloxamat wurden zusammen mit 11.6 g (135 mmol) 3-Methyl-2-butanon in 50ml trockenem THF vorgelegt. 31.8 g (284 mmol) Kalium-tert-butanolat wurden in 200 ml trockenem THF suspendiert und in 40 Minuten zur Reaktionsmischung getropft. Anschliessend wurde über Nacht bei Raumtemperatur gerührt. Man gab 29.2 g (486 mmol) Eisessig zu und filtrierte die Suspension. Der abfiltrierte Feststoff wurde noch mit 300 ml Dichlormethan nachgewaschen und mit dem Filtrat vereint. Die organische Phase wurde am Rotationsverdampfer zur Trockene eingeengt. Das zurückbleibende violette Öl wurde in 67 ml 3 M NaOH-Lösung aufgenommen, der pH-Wert lag bei 12. Man extrahierte die basische wässrige Phase viermal mit je 100 ml Diethylether. Danach wurde der pH-Wert der wässrigen Phase mit konz. Salzsäure auf 1-2 eingestellt. Man extrahierte die wässrige Phase viermal mit einer Ether/Ethylacetat-Mischung (100ml/200ml). Die vereinten organischen Phasen wurden über Natriumsulfat getrocknet und am Rotationsverdampfer zur Trockene eingeengt. Das Rohprodukt wurde säulenchromatographisch (Kieselgel, Ethylacetat/Petrolether = 1/1) gereinigt, die Ausbeute betrug 9.7 g in Form eines gelben Öls.
LC/MS : m/z [Da] = 186.7 $[M+H]^+$, 208.7 $[M+Na]^+$.
Enolform
$^1$H-NMR (CDCl3, 400 MHz): $\delta$ [ppm] = 5.87 (1 H), 3.07 (3H), 2.98 (3H), 2.52 (1 H), 1.16 (3H), 1.14 (3H).
Ketoform
$^1$H-NMR (CDCl3, 400 MHz): $\delta$ [ppm] = 3.96 (2H), 3.09 (3H), 2.96 (3H), 2.65 (1H), 1.12 (3H), 1.10 (3H).

**J. Diethyl 2-oxobutandioat**

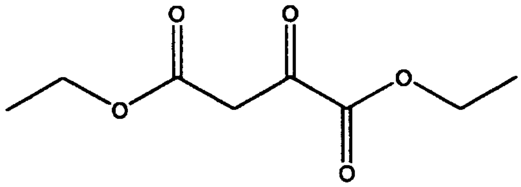

Kommerziell erhältlich: TCI Europe 00073

**K. 5-Methyl-1-(morpholin-4-yl)hexan-1,2,4-trion**

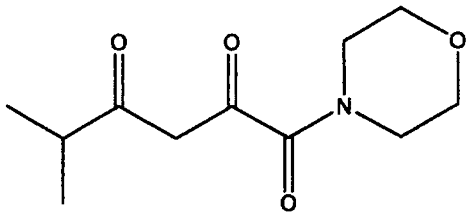

12.7 g (146 mmol) Morpholin und 14.8 g (146 mmol) Triethylamin wurden in 200 ml trockenem Diethylether gelöst und im Eisbad abgekühlt. Man gab 20g (146 mmol) Ethyl chloro-oxoacetat tropfenweise zu und liess auf Raumtemperatur erwärmen. Das ausgefallene Triethylamin-hydrochlorid wurde abfiltriert und das Filtrat am Rotationsverdampfer zur Trockene eingeengt. Von dem zurückgebliebenen gelben Öl wurden 21.9 g zusammen mit 10.1 g (117mmol) 3-Methyl-2-butanon in 40ml trockenem THF vorgelegt. 27.6 g (245 mmol) Kalium-tert-butanolat wurden in 200 ml trockenem THF suspendiert und in 40 Minuten zur Reaktionsmischung getropft. Anschliessend wurde noch 3 Stunden bei Raumtemperatur gerührt. Man gab 25.3 g (421 mmol) Eisessig zu und filtrierte die Suspension. Der abfiltrierte Feststoff wurde noch mit 300 ml Dichlormethan nachgewaschen und mit dem Filtrat vereint. Die organische Phase wurde mit 250 ml 5%iger Natriumhydrogencarbonat-Lösung und 100 ml gesättigter NaCl-Lösung gewaschen. Man trocknete die organische Phase über Natriumsulfat und engte am Rotationsverdampfer zur Trockene ein. Das Rohprodukt wurde säulenchromatographisch (Kieselgel, Ethylacetat/Petrolether = 1/3) gereinigt, die Ausbeute betrug 7.1 g in Form eines klaren Öls.

IR (in Substanz, $cm^{-1}$): 2971, 2929, 2859, 1702, 1641, 1601, 1461, 1439, 1386, 1364, 1328, 1274, 1256, 1204, 1156, 1112, 1070, 1026, 951, 915, 873, 842, 821, 780, 748, 655.

Enolform

$^1$H-NMR (CDCl3, 400 MHz): $\delta$ [ppm] = 5.88 (1H), 3.7-3.5 (8H), 2.50 (1H), 1.14 (3H), 1.12 (3H).

Ketoform

$^1$H-NMR (CDCl3, 400 MHz): $\delta$ [ppm] = 3.94 (2H), 3.7-3.5 (8H), 2.61 (1 H), 1.09 (3H), 1.08 (3H).

**L. 1-(Morpholin-4-yl)pentan-1,2,4-trion**

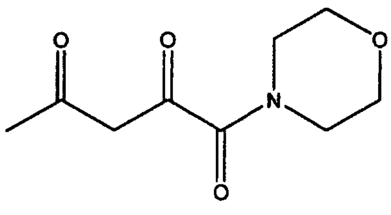

26.1 g (300 mmol) Morpholin und 30.4 g (300 mmol) Triethylamin wurden in 300 ml trockenem Diethylether gelöst und im Eisbad abgekühlt. Man gab 41.0g (300 mmol) Ethyl chlorooxoacetat tropfenweise zu und liess auf Raumtemperatur erwärmen. Das ausgefallene Triethylaminhydrochlorid wurde abfiltriert und das Filtrat am Rotationsverdampfer zur Trockene eingeengt.

Von dem zurückgebliebenen gelben Öl wurden 40.1 g zusammen mit 12.4 g (214 mmol) Aceton in 80ml trockenem THF vorgelegt. 50.4 g (449 mmol) Kalium-tert-butanolat wurden in 200 ml trockenem THF suspendiert und in 40 Minuten zur Reaktionsmischung getropft. Anschliessend wurde noch 3 Stunden bei Raumtemperatur gerührt. Man gab 46.3 g (770 mmol) Eisessig zu und filtrierte die Suspension. Der abfiltrierte Feststoff wurde noch mit 300 ml Dichlormethan nachgewaschen und mit dem Filtrat vereint. Die organische Phase wurde mit 250 ml 5%iger Natriumhydrogencarbonat-Lösung und 100 ml gesättigter NaCl-Lösung gewaschen. Man trocknete die organische Phase über Natriumsulfat und engte am Rotationsverdampfer zur Trockene ein. Das Rohprodukt wurde säulenchromatographisch (Kieselgel, Ethylacetat) gereinigt und anschliessend aus Diethylether kristallisiert. Die Ausbeute betrug 28.9 g in Form hellbrauner Kristalle.
IR (in Substanz, $cm^{-1}$): 3092, 3001, 2969, 2921, 2901, 2858, 2774, 2720, 1615, 1485, 1426, 1371, 1304, 1269, 1214, 1151, 1107, 1067, 1031, 1004, 950, 939, 914, 847, 824, 800, 742, 660.
Enolform:

$^1$H-NMR (CDCl3, 400 MHz): δ [ppm] = 5.93 (1H), 3.8-3.6 (8H), 2.15 (3H).

Ketoform

$^1$H-NMR (CDCl3, 400 MHz): δ [ppm] = 3.98 (2H), 3.8-3.6 (8H), 2.28 (3H).

**M. 1-(Pyrrolidin-1-yl)pentan-1,2,4-trion**

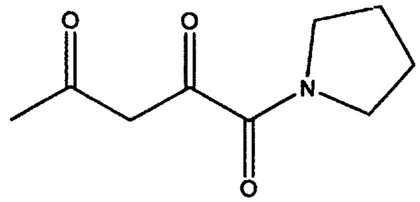

10.4 g (146 mmol) Pyrrolidin und 14.8 g (146 mmol) Triethylamin wurden in 200 ml trockenem Diethylether gelöst und im Eisbad abgekühlt. Man gab 20g (146 mmol) Ethyl chlorooxoacetat tropfenweise zu und liess auf Raumtemperatur erwärmen. Das ausgefallene Triethylamin-hydrochlorid wurde abfiltriert und das Filtrat am Rotationsverdampfer zur Trockene eingeengt.
Von dem zurückgebliebenen gelben Öl wurden 15.1 g zusammen mit 5.1 g (88 mmol) Aceton in 40ml trockenem THF vorgelegt. 20.8 g (185 mmol) Kalium-tert-butanolat wurden in 200 ml trockenem THF suspendiert und in 40 Minuten zur Reaktionsmischung getropft. Anschliessend wurde noch 3 Stunden bei Raumtemperatur gerührt. Man gab 19.0 g (317 mmol) Eisessig zu und filtrierte die Suspension. Der abfiltrierte Feststoff wurde noch mit 300 ml Dichlormethan nachgewaschen und mit dem Filtrat vereint. Die organische Phase wurde mit 250 ml 5%iger Natriumhydrogencarbonat-Lösung und 100 ml gesättigter NaCl-Lösung gewaschen. Man trocknete die organische Phase über Natriumsulfat und engte am Rotationsverdampfer zur Trockene ein. Das Rohprodukt wurde säulenchromatographisch (Kieselgel, Ethylacetat) gereinigt, die Ausbeute betrug 8.14 g in Form eines Feststoffs.
IR (in Substanz, $cm^{-1}$): 3107, 2981, 2956, 2924, 2873, 1594, 1469, 1405, 1336, 1294, 1232, 1184, 1156, 1133, 1111, 1031, 1004, 982, 967, 917, 870, 844, 874, 710.
Enolform
$^1$H-NMR (CDCl3, 400 MHz): δ [ppm] = 6.18 (1H), 3.74 (2H), 3.56 (2H), 2.17 (3H), 2.0-1.85 (4H).
Ketoform
$^1$H-NMR (CDCl3, 400 MHz): δ [ppm] = 3.95 (2H), 3.70 (2H), 3.50 (2H), 2.29 (3H), 2.0-1.85 (4H).

**N. 1-(4-Methoxypiperidin-1-yl)pentan-1,2,4-trion**

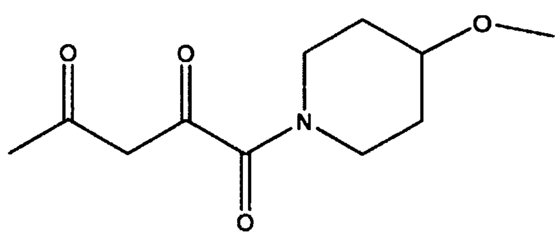

9.79 g (85.0 mmol) 4-Methoxypiperidin und 8.60 g (85.0 mmol) Triethylamin wurden in 50 ml trockenem Diethylether gelöst und in einem Eisbad gekühlt. Zur gekühlten Lösung wurden 11.6 g (85.0 mmol) Ethyl chlorooxoacetat getropft. Man liess auf Raumtemperatur erwärmen und filtrierte das ausgefallene Triethylaminhydrochlorid ab. Das Filtrat wurde am Rotationsverdampfer zur Trockene eingeengt. Man erhielt 10.8 g Rohprodukt in Form eines dunklen Öls.

Dieses wurde ungereinigt zusammen mit 2.90 g (50.0 mmol) Aceton in 80 ml trockenem THF vorgelegt. 11.8 g (105 mmol) Kalium-tert-butoxid wurden in 200 ml trockenem THF aufgenommen und zur Reaktionsmischung getropft. Man liess 3 Stunden bei Raumtemperatur rühren. Danach wurden innerhalb 5 Minuten 10.8g (180 mmol) Eisessig zugegeben und die entstandene Suspension filtriert. Der Filterkuchen wurde mit 200ml Dichlormethan nachgewaschen. Die vereinten organischen Phasen wurden mit 250 ml 5%iger Natriumhydrogencarbonat-Lösung und 100 ml gesättigter Natriumchlorid-Lösung gewaschen, über Natriumsulfat getrocknet und am Rotationsverdampfer zur Trockene eingeengt.

Das Rohprodukt wurde säulenchromatographisch (Kieselgel, Ethylacetat/Petrolether = 3/1) gereinigt, die Ausbeute betrug 3.8 g in Form eines gelben Öls.
IR (in Substanz, $cm^{-1}$): 2931, 2826, 1708, 1637, 1442, 1362, 1309, 1273, 1245, 1205, 1140, 1094, 1076, 1022, 938, 889, 821, 785, 666.
$^1$H-NMR (DMSO, 400 MHz): ö [ppm] = 5.7 (1H), 3.7 (1H), 3.5 (2H), 3.3 (3H), 3.2 (2H), 2.1 (3H), 1.7 (2H), 1.4 (2H).

**O. *N*-(2-Hydroxyethyl)-*N*-methyl-2,4-dioxopentanamid**

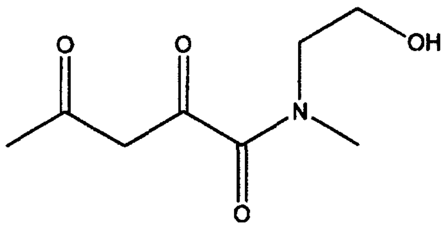

9.98 g (50.0 mmol) Kupferacetat-Monohydrat wurden in 80ml Ethanol suspendiert und auf 50°C erwärmt. Man gab 15.8 g (100 mmol) Ethyl 2,4-dioxovalerat zu und erwärmte auf 60°C. Anschliessend gab man 520ml Ethanol zu und rührte für 2 Stunden bei 60°C. Anschliessend filtrierte man den entstandenen Kupferkomplex ab. Man erhielt nach Trocknen 17.8 g des Kupferkomplexes in Form eines feinen, faserigen Feststoffes.
17.8 g (47.0 mmol) dieses Kupferkomplexes und 21.2 g (283 mmol) 2-Methylaminoethanol wurden in 300 ml Acetonitril gelöst. Zur Entfernung des entstandenen Ethanols, engte man die Reaktionslösung über 4 Stunden bei 50°C und 30 mbar langsam ein. Nach 4 Stunden wurde die Reaktionslösung vollständig zur Trockene eingeengt, man erhielt das Rohprodukt in Form eines dunkelgrünen Öls. Zur Aufarbeitung nahm man das Rohprodukt in 200 ml Chloroform auf und gab 125 ml 30%ige Schwefelsäure zur Zersetzung des Kupferkomplexes zu. Die Phasen wurden getrennt und die wässrige Phase noch zweimal mit 200 ml Chloroform extrahiert. Die vereinten organischen Phasen wurden über Natriumsulfat getrocknet und zur Trockene eingeengt. Man erhielt das Produkt in Form eines gelblichen Öls, das spontan in Halbacetalform kristallisierte.
Halbacetalform:

IR (in Substanz, $cm^{-1}$): 3225, 3012, 2978, 2961, 2921, 1718, 1640, 1514, 1449, 1424, 1401, 1361, 1343, 1264, 1217, 1149, 1166, 1129, 1104, 1067, 1058, 957, 923, 902, 858, 800, 767, 702, 662.

Halbacetalform:

$^1$H-NMR (DMSO, 400 MHz): $\delta$ [ppm] = 6.78 (1 H), 4.10 (1H), 3.65 (1H), 3.50 (1H), 3.15 (1 H), 3.08 (1 H), 2.82 (3H), 2.76 (1 H), 2.03 (3H).

**PHARMAKOLOGISCHE DATEN DER EISEN-KOMPLEXVERBINDUNGEN:**

**Eisen-Utilisation**

**Testmethode:**

Die hervorragenden Fe-Utilisationen, die durch die erfindungsgemäßen Fe-Komplexe, erreicht werden können,

wurden durch folgendes Mäuse-Modell gemessen.

Männliche NMRI (SPF)-Mäuse (ca. 3 Wochen alt) wurden ca. 3 Wochen mit eisenarmer Diät (ca. 5 ppm Eisen) gefüttert. Dann wurde ihnen während 2 mal 5 Tagen mit einer Unterbrechung von 2 Tagen (Tage 1 - 5 und 8 - 12) die Eisenkomplexe mittels Schlundsonde verabreicht (2 mg Eisen/kg Körpergewicht/Tag). Die Utilisation am Tag 15 wurde berechnet aus der Hämoglobinzunahme und der Körpergewichtszunahme nach der Formel

$$\text{Utilisation (\%)} = \frac{\Delta \text{Eisenutilisation} * 100}{\text{Fe Dos.}} = \frac{(\text{Fe ut.} - \text{Fe ut.Control}) * 100}{\text{Fe Dos.}}$$

$$= [(Hb_{2(3)} \cdot BW_{9(14)} - Hb_1 \cdot BW_4) \cdot 0{,}07 \cdot 0{,}0034 - (Hb_{2(3)\,Control} \cdot BW_{9(14)\,Control} - Hb_{1\,Control} \cdot BW_{4\,Control}) \cdot 0{,}07 \cdot 0{,}0034)] \cdot 100/\ Fe\ Dos.$$

$$= [(Hb_{2(3)} \cdot BW_{9(14)} - Hb_1 \cdot BW_4) \cdot 0{,}000238 - (Hb_{2(3)\,Control} \cdot BW_{9(14)\,Control} - Hb_{1\,Control} \cdot BW_{4\,Control}) \cdot 0{,}000238] \cdot 100\ /\ Fe\ Dos.$$

$$= (Hb_{2(3)} \cdot BW_{9(14)} - Hb_1 \cdot BW_4 - Hb_{2(3)\,Control} \cdot BW_{9(14)\,Control} + Hb_{1\,Control} \cdot BW_{4\,Control}) \cdot 0{,}0238\ /\ Fe\ Dos.$$

| | |
|---|---|
| 0.07 = | Faktor für 70 ml Blut per kg Körpergewicht (BW) |
| 0.0034 = | Faktor für 0,0034 g Fe/g Hb |
| $Hb_1$ = | Hämoglobin-Wert (g/l) am Tag 1 |
| $Hb_{2(3)}$ = | Hämoglobin-Wert (g/l) am Tag 8 (or 15) |
| $BW_4$ = | Körpergewicht (g) am Tag 1 |
| $BW_{9(14)}$ = | Körpergewicht (g) am Tag 8 (or 15) |
| $Hb_{1\,Control}$ = | Durchschnitts-Hämoglobinwert (g/l) am Tag 1 in der Kontrollgruppe, |
| $Hb_{2(3)\,Control}$ = | Durchschnitts-Hämoglobinwert (g/l) am Tag 8 (oder 15) in der Kontrollgruppe, |
| $BW_{4\,Control}$ = | Durchschnittskörpergewicht (g) am Tag 1 in der Kontrollgruppe, |
| $BW_{9(14)\,Control}$ = | Durchschnittskörpergewicht (g) am Tag 8 (oder 15) in der Kontrollgruppe, |
| Fe Dos. = | Gesamtes verabreichtes Eisen (mg Fe) während 5 oder 10 Tage, Fe ut. = ($Hb_{2(3)}$ $BW_{9(14)}$- $Hb_1$ * $BW_4$) * 0.07 * 0.0034 (mg Fe) |
| Δ Utilisation = | Fe tot. utilisiert (Untersuchte Gruppe) - Fe ut. Kontrollgruppe, utilisiert aus Nahrung, (mg Fe) |

Tabelle:

| Beispiel-Nr. | Utilisation n 15 d (abs. %) |
|---|---|
| 1 | 106 |
| 2 | 94 |
| 3 | 82 |
| 4 | Nicht bestimmt |
| 5 | 59 |
| 6 | 65 |
| 7 | Nicht bestimmt |
| 8 | 72 |
| 9 | 89 |
| 10 | Nicht bestimmt |
| 11 | 51 |
| 12 | 75 |

Die gemessenen Eisen-Utilisationswerte stellen einen wichtigen Parameter im Hinblick auf die Indikation der Behandlung von Eisenmangelerscheinungen und Eisenmangelanämien dar, denn dieser Parameter spiegelt nicht nur die Eisenaufnahme, sondern auch den Zusammenhang zwischen Körpergewicht und Eisenaufnahme wieder, was gerade bei der Verwendung von heranwachsenden Tieren im Tiermodell wichtig ist. Würde man nur die Hämoglobin-Werte betrachten, die ein Maß für das wirklich aufgenommene und verwendete Eisen darstellen, würde man den auf dem Wachstum der Tiere beruhenden Anteil unberücksichtigt lassen. Somit stellt die Eisen-Utilisation eine genauere Meßgröße dar, obwohl Eisenutilisation und Hämoglobinwerte meist miteinander korrelieren. Eine Betrachtung des reinen Eisen-Serumspiegels, welche ebenfalls messbar wäre, kommt weniger in Betracht, da zwar eine Aussage über die Menge Eisen getroffen wird, die in den Körper gelangt, aber nicht darüber, wieviel der Körper davon auch nutzen kann.

Die Testergebnisse zeigen, dass die erfindungsgemäßen Eisenkomplexverbindungen über eine hervorragende Eisenutilisation verfügen, so dass sie als Mittel zur Behandlung von Eisenmangelanämien und der damit verbundenen Symptome geeignet sind.

**Toxizität**

**Toxizitätsvergleich mit $Fe(Acac)_3$**

Die Toxizität der erfindungsgemäßen Eisen-Komplexverbindung wurde mit der von Tris(acetylacetonat)-eisen verglichen. Dabei wurde 6 Sprague-Dawley Ratten einmalig eine orale Dosis der Eisen-Komplexverbindung von Beispiel 3 (1400 mg/kg) und von Beispiel 1 (1600 mg/kg), entsprechend jeweils ca. 170 mg Eisen, verabreicht. Nach 24 Stunden waren alle 6 Versuchstiere noch lebendig und zeigten keinerlei toxikologische Befunde. Demgegenüber verstarben bei Verabreichung von Tris(acetylacetonat)-eisen innerhalb von 24 Stunden 6 von 10 Versuchstieren (Lit. 1 s. Tabelle I). Die folgende Tabelle fasst die Ergebnisse zusammen:

**Test: Orale Applikation bei Sprague-Dawley Ratten / Einmalgabe / Beobachtung bis 24 h nach Gabe**

| | $Fe(Acac)_3$ (Lit.1) | Bsp.3 | Bsp.1 |
|---|---|---|---|
| Verabreichte Menge Eisen-Komplex / [mg/kg] | 1000 | 1400 | 1600 |
| Verabreichte Menge Eisen / [mg/kg] | 160 | 170 | 170 |
| Anzahl der Tiere | 10 | 6 | 6 |
| Anzahl der toten Tiere nach 24 h | 6 | 0 | 0 |

Die Ergebnisse zeigen, dass die erfindungsgemäßen Eisen-Komplexverbindungen im Unterschied zum stark toxischen Tris(acetylacetonat)-eisen praktisch keine toxikologischen Befunde zeigen.

(Lit.1: London, J. E.; Smith, D. M. Preliminary toxicological study of ferric acetylacetonate. Report (1983), (LA-9627-MS; Order No. DE83007117), 7 pp. CAN 99:48652 AN 1983:448652 CAPLUS)

**Herstellungsbeispiele:**

**Beispiel 1**

**Tris-(ethyl 2,4-dioxopentanoat)-eisen(III)-Komplex**

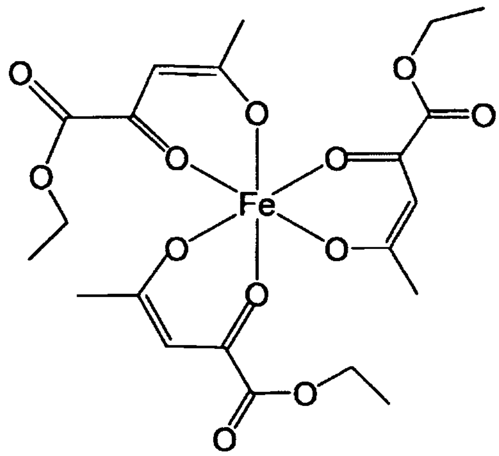

25.0 g (158 mmol) Ethyl-2,4-dioxopentanoat wurden in 50 ml EtOH 92% vorgelegt und 24.5 g wässrige Eisen(III)chlorid-Lösung (12 % m/m Fe, 53 mmol) innerhalb von ca. 4 min bei 25 ± 5 °C zugegeben (schwach exotherm, Kühlung mit Eisbad). Anschliessend wurden unter Rühren 21.8 g NaOH (30 % m/m, 164 mmol) innerhalb von ca. 15 min. bei 25 ± 5 °C zugegeben (Kühlung mit Eisbad). Nach 2 h Reaktionszeit bei 25 ± 5 °C wurden unter Rühren 240 ml Wasser zugegeben. Die Suspension wurde für 2 h bei 0 - 5 °C gerührt, filtriert und der Filterkuchen mit 2 x 20 ml Wasser gewaschen. Das Produkt wird für 48 h bei 50°C im Feinvakuum getrocknet. Ausbeute: 23.0 g roter Feststoff.
IR (in Substanz, $cm^{-1}$): 3129, 2986, 1733, 1587, 1509, 1415, 1360, 1251, 1215, 1174, 1141, 1096, 1005, 948, 911, 863, 828, 793, 760, 634.
Elementaranalyse: C 48.2%, H 5.0%.
Fe-Gehalt: 10.4 % [m/m].

**Beispiel 2**

**Tris-(methyl 2,4-dioxopentanoat)-eisen(III)-Komplex**

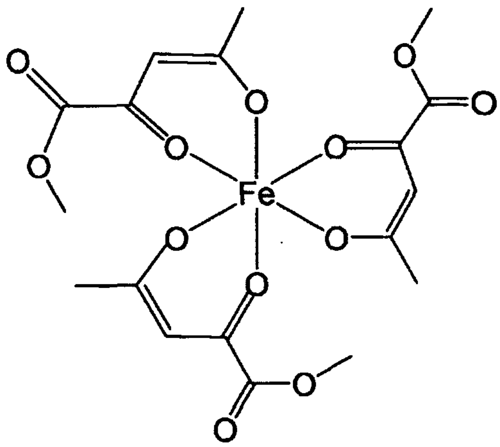

Es wurden 6.49 g (40 mmol) Eisen(III)-chlorid in 100 ml Ethanol gelöst und tropfenweise zu einer Lösung aus 17.30 g (120 mmol) Methyl-2,4-dioxopentanoat in 100 ml Ethanol gegeben. Während der Zugabe verfärbte sich die Lösung von gelb nach violett. Die Lösung wurde 30 min bei RT gerührt und anschliessend wurden 16.80 g (200 mmol) Natriumhydrogencarbonat zugegeben. Die Mischung wurde noch 1 h bei RT gerührt und dann bis zur Trockene eingeengt. Der Rückstand wurde in 100 ml Wasser (pH 6.5) aufgenommen und der pH-Wert auf 7.6 mit Natriumhydrogencarbonat (ca. 3.36 g, 40 mmol) eingestellt. Die Mischung wurde noch 2 h bei RT nachgerührt, der Niederschlag abfiltriert und bei 45 °C im Vakuumtrockenschrank getrocknet. Anschliessend wurden 17.06 g eines dunkelroten Pulvers erhalten.
IR (in Substanz, $cm^{-1}$): 3461, 3129, 2954, 2924, 2844, 2458, 2384, 2289, 2171, 2107, 1988, 1908, 1738, 1586, 1509, 1407, 1364, 1259, 1222, 1144, 1021, 968, 950, 876, 830, 794, 778, 634.

Elementaranalyse: C 44.3%, H 4.3%.
Fe-Gehalt: 11.12 % [m/m].

**Beispiel 3**

**Tris-(2,4-dioxopentanamid)-eisen(III)-Komplex**

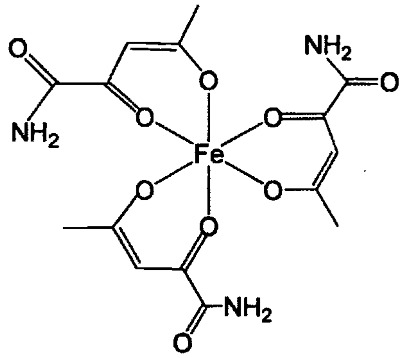

11.0 g (85.0 mmol) 2,4-Dioxopentanamid wurden in 730 ml Ethanol gelöst und auf 40°C erwärmt. 4.60 g (28.3 mmol) Eisen(III)chloride wurden in 46 ml Ethanol gelöst und tropfenweise zur 2,4-Dioxopentanamid-Lösung gegeben. Danach gab man 11.8 ml (85.0 mmol) Triethylamin zu. Die Reaktionslösung wurde zur Trockene eingeengt, in 2,3 l Dichlormethan aufgeschlämmt und filtriert. Der hellrote Feststoff wurde über Nacht bei 50°C im Feinvakuum getrocknet. Man erhielt 6.2g des Produkts.
IR (in Substanz, $cm^{-1}$): 3430, 3293, 3161, 2969, 2927, 2884, 1682, 1587, 1512, 1427, 1356, 1231, 1145, 1091, 1035, 947, 885, 794, 690.
Fe-Gehalt: 12.54 % [m/m].

**Beispiel 4**

**Tris-(2,4-dioxopentansäure)-eisen(III)-Komplex**

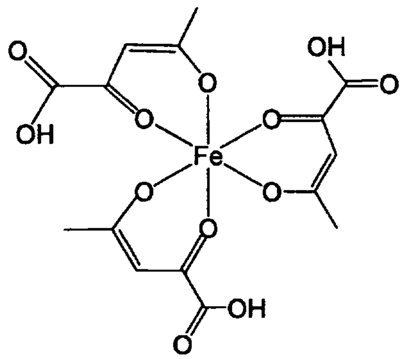

Es wurden 0.83 g (5.1 mmol) Eisen(III)-chlorid in 50 ml Ethanol gelöst und tropfenweise zu einer Lösung aus 2.00 g (15.4 mmol) 2,4-dioxopentansäure in 50 ml Ethanol gegeben. Die Lösung wurde 30 min bei RT gerührt und anschliessend wurden 3.32 g (15.4 mmol) 25 %ige Natriummethylat-Lösung zugegeben. Die Mischung wurde noch 2 h bei RT gerührt und dann bis zur Trockene eingeengt. Der Rückstand wurde bei 50 °C im Vakuumtrockenschrank getrocknet. Es wurden 10.07 g Produkt in Form eines dunkelbraunen Pulvers erhalten.
IR (in Substanz, $cm^{-1}$): 2920, 1713, 1586, 1517, 1358, 1234, 1157, 1010, 947, 909, 788, 725, 626, 589, 537, 495.
Fe-Gehalt: 9.02 % [m/m].

**Beispiel 5**

**Tris-(ethyl oxo(2-oxocyclopentyl)acetat)-eisen(III)-Komplex**

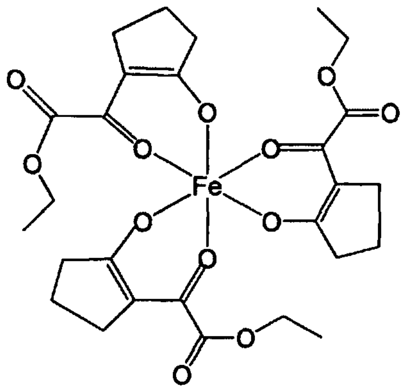

Es wurden 0.81 g (4.99 mmol) Eisen(III)-chlorid in 20 ml Ethanol gelöst. 2.68 g (14.5 mmol) Ethyl oxo(2-oxocyclopentyl)acetat wurden in 20 ml Ethanol gelöst und zur Eisen(III)-chlorid-Lösung gegeben. Während der Zugabe verfärbte sich die Lösung von gelb nach dunkelrot. Die Lösung wurde 5 min bei Raumtemperatur gerührt und anschliessend wurden 1.26 g (15.0 mmol) Natriumhydrogencarbonat zugegeben. Die Mischung wurde noch 1 h bei RT gerührt und dann bis zur Trockene eingeengt. Der Rückstand wurde in 100 ml Wasser aufgenommen, zwei Stunden bei RT nachgerührt (pH bei 8.0) und mit Ethylacetat extrahiert. Das Ethylacetat wurde am Rotationsverdampfer vollständig entfernt. Der Rückstand wurde dreimal mit 100ml n-Hexan ausgezogen und die vereinten n-Hexanfraktionen zur Trockene eingeengt. Es wurden 2.6 g eines dunkelroten Feststoffs erhalten.
IR (in Substanz, $cm^{-1}$): 2964, 1725, 1672, 1597, 1551, 1488, 1390, 1365, 1212, 1118, 1015,917,861,839,803,771,705,634.
Fe-Gehalt: 9.25 % [m/m].

**Beispiel 6**

**Tris-(ethyl oxo(2-oxocyclohexyl)acetat)-eisen(III)-Komplex**

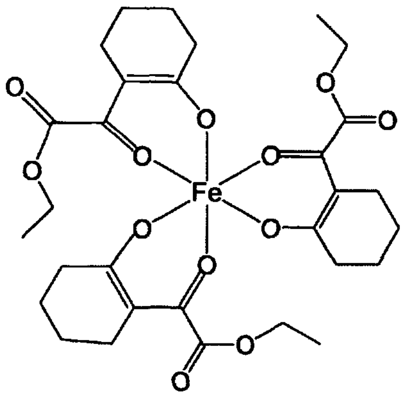

Es wurden 0.81 g (4.99 mmol) Eisen(III)-chlorid in 20 ml Ethanol gelöst, 2.88 g (14.5 mmol) Ethyl oxo(2-oxocyclohexyl)acetat zugegeben und die Reaktionsmischung mit weiteren 20 ml Ethanol verdünnt. Während der Zugabe verfärbte sich die Lösung von orange nach dunkelrot. Die Lösung wurde 10 min bei Raumtemperatur gerührt und anschliessend wurden 3.23 g (15.0 mmol) Natriummethanolat-Lösung (25%) zugegeben. Die Mischung wurde noch 1 h bei RT gerührt und bis zur Trockene eingeengt. Der Rückstand wurde mit 90 ml tert-Butylmethylether versetzt und über Nacht bei Raumtemperatur gerührt. Man filtrierte den Feststoff ab und trocknete diesen im Feinvakuum. Es wurden

2.8 g eines dunkelroten Feststoffs erhalten.
IR (in Substanz, $cm^{-1}$): 2976, 2932, 2860, 1736, 1654, 1581, 1482, 1447, 1413, 1379, 1363, 1312, 1297, 1242, 1210, 1164, 1080, 1061, 1017, 972, 919, 827, 771, 726, 669.
Elementaranalyse: C 54.7%, H 6.9%.
Fe-Gehalt: 8.27 % [m/m].

**Beispiel 7**

**Tris-(5,5-dimethyl-1-(piperidin-1-yl)hexan-1,2,4-trion)-eisen(III)-Komplex**

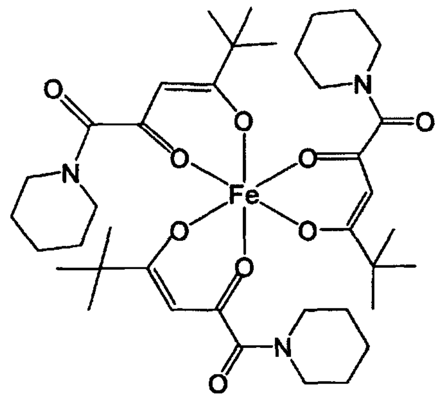

Es wurden 3.50 g (1.39 mmol) Eisen(III)-ethoxid-Lösung (Ethanol, 2.22%) in 8 ml trockenem Ethanol aufgenommen. 1.00 g (4.18 mmol) 5,5-Dimethyl-1-(piperidin-1-yl)hexan-1,2,4-trion wurden zugegeben und die Reaktionslösung über Nacht gerührt. Während der Zugabe verfärbte sich die Lösung von hellrot nach weinrot. Das Lösungsmittel wurde am Rotationsverdampfer vollständig entfernt. Der Rückstand wurde in Ethylacetat vollständig gelöst und nochmals zur Trockene eingeengt. Der Rückstand wurde über Nacht bei 50°C im Feinvakuum getrocknet. Es wurden 1.21 g eines dunkelroten Feststoffs erhalten.
IR (in Substanz, $cm^{-1}$): 3450, 2936, 2861, 1637, 1550, 1443, 1399, 1387, 1359, 1293, 1254, 1235, 1179, 1154, 1131, 1049, 1025, 1010, 964, 928, 895, 843, 824, 810, 780, 706,644.
Fe-Gehalt: 7.5 % [m/m].

**Beispiel** 8

**Tris-(N,N,5,5-tetramethyl-2,4-dioxohexanamid)-eisen(III)-Komplex**

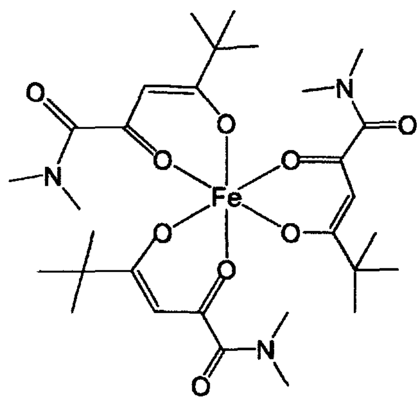

Es wurden 3.88 g (1.54 mmol) Eisen(III)-ethoxid-Lösung (Ethanol, 2.22%) in 8 ml trockenem Ethanol aufgenommen. 0.92 g (4.62 mmol) N,N,5,5-tetramethyl-2,4-dioxohexanamid wurden zugegeben und die Reaktionslösung

über Nacht gerührt. Das Lösungsmittel wurde am Rotationsverdampfer vollständig entfernt. Der Rückstand wurde über Nacht bei 50°C im Feinvakuum getrocknet. Es wurden 1.1 g eines dunkelroten Öls erhalten.
IR (in Substanz, $cm^{-1}$): 2965, 2869, 1644, 1551, 1489, 1382, 1355, 1287, 1257, 1228, 1202, 1153, 1108, 1062, 1022, 969, 934, 840, 809, 780, 734, 706, 644.
Fe-Gehalt: 8.7 % [m/m].

**Beispiel 9**

**Tris-(N,N,5,-trimethyl-2,4-dioxohexanamid)-eisen(III)-Komplex**

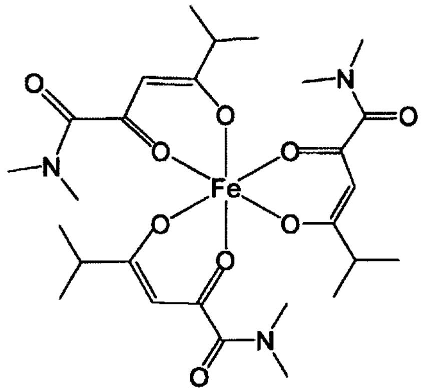

0.29 g (1.80 mmol) Eisen(III)-chlorid wurden in 10 ml trockenem THF gelöst. 1.00 g (5.40 mmol) N,N,5-trimethyl-2,4-dioxohexanamid wurden in 1 ml trockenem THF gelöst und zur Eisen(III)-chlorid-Lösung gegeben. 0.56 g (5.58 mmol) Triethylamin wurden in 2 ml trockenem THF aufgenommen und zur Reaktionsmischung getropft. Man rührte 15 Minuten bei Raumtemperatur. Man gab 100 ml Diethylether zu und filtrierte die entstandene Suspension ab. Das Filtrat wurde am Rotationsverdampfer zur Trockene eingeengt, in 100 ml Diethylether aufgenommen und nochmals filtriert. Das Filtrat wurde am Rotationsverdampfer zur Trockene eingeengt und der Rückstand über Nacht bei 50°C im Feinvakuum getrocknet. Man erhielt 0.84g eines roten, glasartigen Feststoffs.
IR (in Substanz, $cm^{-1}$): 2968, 2932, 2873, 1649, 1555, 1491, 1389, 1338, 1261, 1224, 1149, 1112, 1094, 1060, 966, 944, 883, 814, 796, 784, 752, 719, 677, 654.
Fe-Gehalt: 7.48 % [m/m].

**Beispiel 10**

**Tris-(diethyl 2-oxobutandioat)-eisen(III)-Komplex**

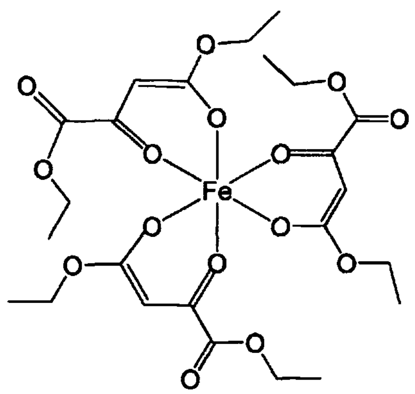

0.65 g (4.00 mmol) Eisen(III)chlorid wurden in 25 ml trockenem THF gelöst und 2.26 g (12.0 mmol) Diethyl 2-oxobutandioat zugegeben. Nach 30min Rührzeit gab man tropfenweise 1.21 g (12.0 mmol) Triethylamin gelöst in 12.5ml trockenem THF zur blauen Reaktionslösung und liess für weitere 30min bei Raumtemperatur rühren. Man verdünnte die nun rote Reaktionslösung mit 30 ml trockenem THF. Es wurden anschliessend 12.5 g 6.4%ige Natriummethanolat-Lösung (11.8 mmol) verdünnt in 12.5 ml trockenem THF zugetropft. Das ausgefallene Salz wurde abfiltriert und das Filtrat am Rotationsverdampfer bis zur Trockene eingeengt. Der Rückstand wurde bei 50°C im Feinvakuum getrocknet. Das Rohprodukt wurde dann in 200 ml Diethylether aufgenommen, filtriert und das Filtrat am Rotationsverdampfer zur Trockene eingeengt. Man erhielt 2.3 g eines tiefroten Harzes.
IR (in Substanz, $cm^{-1}$): 2983, 2938, 1790, 1726, 1597, 1537, 1476, 1446, 1389, 1367, 1260, 1229, 1131, 1094, 1032, 989, 946, 859, 822, 785, 685.
Fe-Gehalt: 8.93% [m/m].

**Beispiel 11**

**Tris-(5-Methyl-1-(morpholin-4-yl)hexan-1,2,4-trion)-eisen(III)-Komplex**

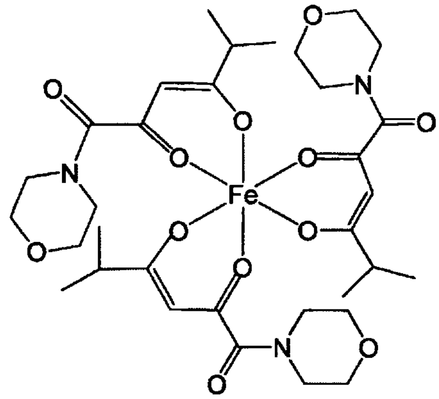

0.24 g (1.48 mmol) Eisen(III)-chlorid wurden mit 0.60g (4.40 mmol) Natriumacetat in 10 ml Wasser gelöst. 1.00 g (4.40 mmol) 5-Methyl-1-(morpholin-4-yl)hexan-1,2,4-trion zugegeben und die Lösung 5 Minuten gerührt. 0.37 g (4.49 mmol) Natriumhydrogencarbonat wurden in einer minimalen Menge Wasser gelöst und zur Reaktionsmischung getropft. Man extrahierte die Reaktionsmischung mit Ethylacetat, trocknete über Natriumsulfat und engte am Rotationsverdampfer zur Trockene ein. Der Rückstand wurde über Nacht bei 50°C im Feinvakuum getrocknet. Man erhielt 0.8 g eines roten, glasartigen Feststoffs.
IR (in Substanz, $cm^{-1}$): 2968, 2927, 2858, 1643, 1556, 1520, 1434, 1406, 1359, 1298, 1274, 1252, 1202, 1165, 1150, 1111, 1065, 1028, 965, 944, 916, 878, 842, 815, 783, 748.
Fe-Gehalt: 7.48 % [m/m].

**Beispiel 12**

**Tris-(1-(Morpholin-4-yl)pentan-1,2,4-trion)-eisen(III)-Komplex**

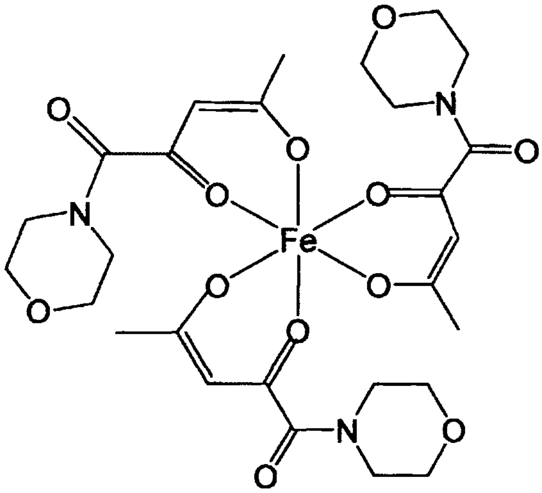

4.12 g (21.3 mmol) Eisen(III)-acetat wurden mit 13.1g (66.0 mmol) 1-(Morpholin-4-yl)pentan-1,2,4-trion in 190 ml Ethylacetat suspendiert und für 60 Minuten bei 60°C gerührt. Man filtriert anschliessend die unlöslichen Bestandteile ab und kristallisierte das Produkt aus der Reaktionsmischung bei 4°C aus. Der auskristallisierte Niederschlag wurde abfiltriert, in Aceton gelöst und am Rotationsverdampfer erneut zur Trockene eingeengt. Der Rückstand wurde über Nacht bei 50°C im Feinvakuum getrocknet. Man erhielt 11.0 g eines roten Feststoffs.
IR (in Substanz, $cm^{-1}$): 2979, 2918, 2855, 1644, 1559, 1523, 1437, 1390, 1357, 1300, 1283, 1269, 1248, 1224, 1169, 1111, 1067, 1036, 953, 919, 852, 823, 807, 790, 760, 669.
Fe-Gehalt: 8.05 % [m/m].

**Beispiel 13**

**Tris-(1-(Pyrrolidin-1-yl)pentan-1,2,4-trion)-eisen(III)-Komplex**

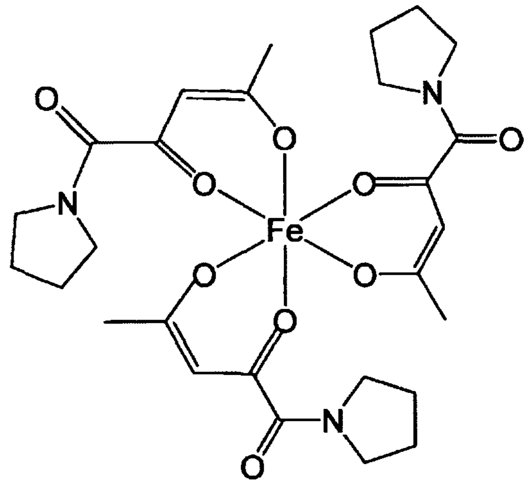

0.54 g (3.30 mmol) Eisen(III)-chlorid wurden mit 1.36g (10.0 mmol) Natriumacetat in 20 ml Wasser gelöst. 1.83 g (10.0 mmol) 1-(Pyrrolidin-1-yl)pentane-1,2,4-trion wurden in 40 ml Ethylacetat gelöst und zur Reaktionsmischung gegeben. Unter Rühren wurde der pH-Wert der wässrigen Phase mit gesättigter Natriumhydrogencarbonat-Lösung auf 6.5 eingestellt. Nach Abtrennen des Ethylacetats extrahierte man die wässrige Phase dreimal mit 200ml Ethylacetat, trocknete über Natriumsulfat und engte am Rotationsverdampfer zur Trockene ein. Man erhielt 1.5 g eines roten Feststoffs.
IR (in Substanz, $cm^{-1}$): 2969, 2877, 1701, 1633, 1562, 1520, 1427, 1361, 1250, 1225, 1187, 1159, 1113, 1014, 980, 951, 917, 870, 824, 783, 718.
Fe-Gehalt: 8.19 % [m/m].

**Beispiel 14**

**Tris-(1-(4-Methoxypiperidin-1-yl)pentan-1,2,4-trion)-eisen(III)-Komplex**

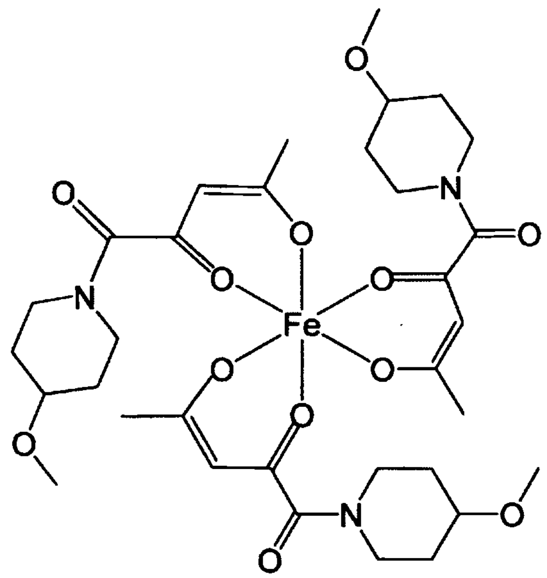

0.56 g (2.90 mmol) Eisen(III)-acetat wurden mit 2.04 g (9.00 mmol) 1-(4-methoxypiperidin-1-yl)pentan-1,2,4-trion in 30 ml Ethylacetat suspendiert und für 60 Minuten bei 60°C gerührt. Man filtriert anschliessend die unlöslichen Bestandteile ab und engte das Filtrat am Rotationsverdampfer zur Trockene ein. Man gab 80 ml Toluol zu und engte erneut am Rotationsverdampfer zur Trockene ein. Diese Prozedur wiederholte man dreimal. Der Rückstand wurde über Nacht bei 50°C im Feinvakuum getrocknet. Man erhielt 1.9 g eines roten, amorphen Feststoffs.
IR (in Substanz, $cm^{-1}$): 2930, 2825, 1637, 1561, 1527, 1445, 1393, 1358, 1318, 1282, 1260, 1221, 1185, 1162, 1094, 1075, 1020, 955, 939, 889, 834, 820, 782, 735, 698, 671.
Fe-Gehalt: 7.06 % [m/m].

**Beispiel 15**

**Tris-(*N*-(2-hydroxyethyl)-*N*-methyl-2,4-dioxopentanamid)-eisen(III)-Komplex**

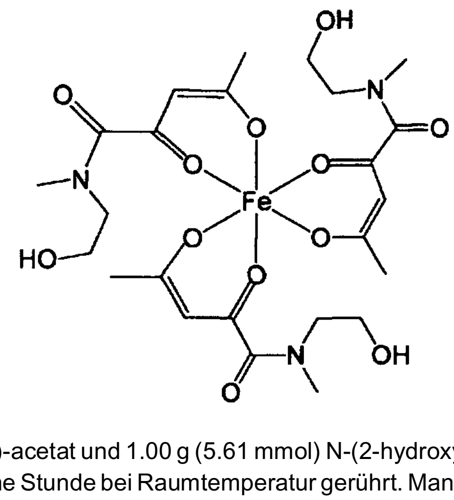

0.26 g (1.62 mmol) Eisen(III)-acetat und 1.00 g (5.61 mmol) N-(2-hydroxyethyl)-N-methyl-2,4-dioxopentanamid wurden in 10ml Wasser gelöst und eine Stunde bei Raumtemperatur gerührt. Man gab 0.59 g (4.35 mmol) Natriumacetat-Trihydrat zu und rührt 10 Minuten bei Raumtemperatur. Danach gab man 0.21 g (2.52 mmol) Natriumhydrogencarbonat

zu und lyophilisierte die Reaktionsmischung unmittelbar danach. Der Rückstand wurde in 50 ml Ethanol aufgenommen und die unlöslichen Salze abfiltriert. Man engte das Filtrat zur Trockene ein und nahm den Rückstand in 50 ml Ethylacetat auf. Man filtrierte weitere unlösliche Salze ab und engte das Filtrat erneut zur Trockene ein. Überschüssiger Ligand wurde durch Kochen mit 50 mL Diethylether entfernt und der zurückgebliebene Komplex im Vakuum getrocknet. Man erhielt 0.8 g Komplex in Form eines roten Feststoffs.
IR (in Substanz, $cm^{-1}$): 3388, 2931, 1626, 1593, 1484, 1389, 1290, 1113, 1048, 1018, 956, 923, 864, 835, 771.
Fe-Gehalt: 7.7 % [m/m].

## Patentansprüche

**1.** Eisen(III)-2,4-dioxo-1-carbonyl-Komplexverbindungen, enthaltend mindestens einen Liganden der Formel (I):

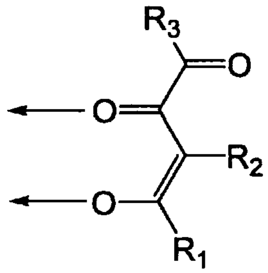

(I)

worin
die Pfeile jeweils eine koordinative Bindung zu einem oder verschiedenen Eisenatomen darstellen, und
$R_1$ ausgewählt wird aus der Gruppe, die besteht aus

- gegebenenfalls substituiertem Alkyl,
- gegebenenfalls substituiertem Alkoxy und
- gegebenenfalls substituiertem Alkoxycarbonyl,

$R_2$ ausgewählt wird aus der Gruppe, die besteht aus

- Wasserstoff,
- gegebenenfalls substituiertem Alkyl und
- Halogen, oder

$R_1$ und $R_2$ gemeinsam mit den Kohlenstoffatomen, an die sie gebunden sind, einen gegebenenfalls substituierten 5- oder 6-gliedrigen Ring bilden, der gegebenenfalls ein oder mehrere Heteroatome aufweisen kann,
$R_3$ ausgewählt wird aus der Gruppe, die besteht aus

- gegebenenfalls substituiertem Alkyl,
- gegebenenfalls substituiertem Alkoxy,
- gegebenenfalls substituiertem Amino und
- Hydroxy,

oder pharmazeutisch verträgliche Salze davon,
zur Anwendung in der Behandlung und Prophylaxe von Eisenmangelerscheinungen und Eisenmangelanämien.

**2.** Eisen(III)-Komplexverbindungen zur Anwendung nach Anspruch 1, worin $R_3$ gegebenenfalls substituiertes Amino ist.

**3.** Eisen(III)-Komplexverbindungen zur Anwendung nach Anspruch 1 oder 2,
worin
$R_1$ aus der Gruppe ausgewählt wird, die besteht aus:

- Alkyl, dass gegebenenfalls durch 1 bis 3 Substituenten substituiert sein kann, die aus der Gruppe ausgewählt sind, die besteht aus: Hydroxy, Alkoxy, Alkoxycarbonyl und Aminocarbonyl,

- Alkoxycarbonyl, dass gegebenenfalls durch 1 bis 3 Substituenten substituiert sein kann, die aus der Gruppe ausgewählt sind, die besteht aus: Hydroxy und Alkoxy, und
- Alkoxy, dass gegebenenfalls durch 1 bis 3 Substituenten substituiert sein kann, die aus der Gruppe ausgewählt sind, die besteht aus: Hydroxy, Alkoxy und Halogen,

$R_2$ aus der Gruppe ausgewählt wird, die besteht aus:

- Wasserstoff,
- Alkyl, dass gegebenenfalls durch 1 bis 3 Substituenten substituiert sein kann, die aus der Gruppe ausgewählt sind, die besteht aus: Hydroxy, Alkoxy, Halogen und Alkoxycarbonyl,
- Halogen, ausgewählt aus Chlor und Fluor,

oder
$R_1$ und $R_2$ gemeinsam mit den Kohlenstoffatomen, an die sie gebunden sind, einen 5- oder 6-gliedrigen Ring bilden, der gegebenenfalls ein oder zwei Heteroatome aufweisen kann, und ein bis drei weitere Substituenten tragen kann, die aus der Gruppe ausgewählt sind, die besteht aus: Hydroxy, Alkoxy, Alkoxycarbonyl und Aminocarbonyl, und
$R_3$ aus der Gruppe ausgewählt wird, die besteht aus:

- Hydroxy,
- Alkyl, dass gegebenenfalls durch 1 bis 3 Substituenten substituiert sein kann, die aus der Gruppe ausgewählt sind, die besteht aus: Hydroxy, Alkoxy und Alkoxycarbonyl,
- Alkoxy, dass gegebenenfalls durch 1 bis 3 Substituenten substituiert sein kann, die aus der Gruppe ausgewählt sind, die besteht aus: Hydroxy, Alkoxy und Alkoxycarbonyl, und
- Amino, dass gegebenenfalls durch 1 bis 3 Substituenten substituiert sein kann, die aus der Gruppe ausgewählt sind, die besteht aus: Alkyl, Hydroxyalkyl, Alkoxy und Alkoxycarbonyl

oder pharmazeutisch verträgliche Salze davon.

**4.** Eisen(III)-Komplexverbindungen zur Anwendung nach Anspruch 1, 2 oder 3, worin
$R_1$ aus der Gruppe ausgewählt wird, die besteht aus:

- Methyl
- iso-Propyl
- tert.-Butyl
- Methoxy
- Ethoxy
- Methoxycarbonyl und
- Ethoxycarbonyl,

$R_2$ aus der Gruppe ausgewählt wird, die besteht aus:

- Wasserstoff
- Methyl und
- Fluor, oder

$R_1$ und $R_2$ bilden zusammen eine Propylen- ($-CH_2-CH_2-CH_2-$) oder Butylen- ($-CH_2-CH_2-CH_2-CH_2-$) Gruppe, und
$R_3$ aus der Gruppe ausgewählt wird, die besteht aus:

- Hydroxy
- Methoxy
- Ethoxy
- Amino
- Dimethylamino
- 3-Propylamino
- 2-Ethylamino
- Morpholino
- Piperidino und
- 4-Hydroxypiperidino oder pharmazeutisch verträgliche Salze davon.

5. Eisen(III)-Komplexverbindungen zur Anwendung nach einem der Ansprüche 1 bis 4, welche die folgende Formel aufweisen:

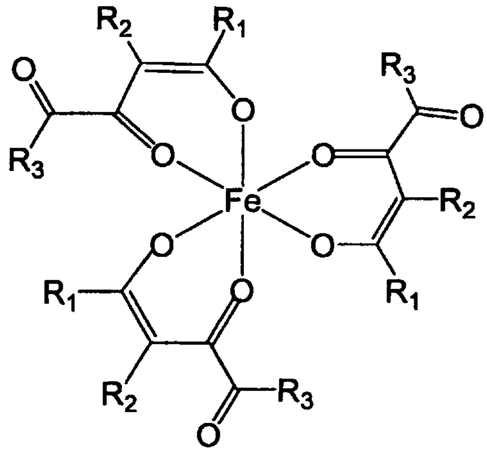

,

worin $R_1$, $R_2$, $R_3$ jeweils gleich oder verschieden sein können und wie oben definiert sind, und pharmazeutisch verträgliche Salze davon.

6. Eisen(III)-Komplexverbindungen zur Anwendung nach einem der Ansprüche 1 bis 5, worin die mit den Eisenmangelerscheinungen und Eisenmangelanämien einhergehenden Symptome einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

7. Eisen(III)-Komplexverbindungen zur Anwendung nach einem der Ansprüche 1 bis 6 zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CKD 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases).

8. Eisen(III)-Komplexverbindungen zur Anwendung nach einem der Ansprüche 1 bis 7 zur oralen Verabreichung.

9. Eisen(III)-Komplexverb9ndungen zur Anwendung nach Anspruch 8, worin eine feste Formulierung, wie Pillen, Tabletten, magensaftresistente Tabletten, Filmtabletten, Schichttabletten, Retardformulierungen, Depotformulierungen, Dragees, Zäpfchen (Suppositorien), Granulat, Mikrokapseln, Mikroformulierungen, Nano-Formulierungen, Kapseln, magensaftresistente Kapseln und Pulver, oder eine flüssige Formulierung, einschließlich einer trinkbaren Formulierung, wie Sirup, Elixier, Lösung, Suspension, Saft, verabreicht wird.

10. Arzneimittel, enthaltend Eisen(III)-Komplexverbindungen wie in einem der Ansprüche 1 bis 5 definiert sowie mindestens einen physiologisch verträglichen Träger bzw. Exzipienten.

11. Zusammensetzung enthaltend Eisen(III)-Komplexverbindungen, wie in einem der Ansprüche 1 bis 5 definiert, in

Kombination mit mindestens einem weiteren Arzneimittel, welches auf den Eisenmetabolismus wirkt.

**Claims**

1. Iron(III)-2,4-dioxo-1-carbonyl complex compounds containing at least one ligand of the formula (I):

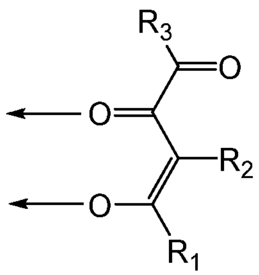

(I)

wherein
the arrows each represent a coordinate bond to one or different iron atoms, $R_1$ is selected from the group consisting of

- optionally substituted alkyl,
- optionally substituted alkoxy and
- optionally substituted alkoxycarbonyl,

$R_2$ is selected from the group consisting of

- hydrogen,
- optionally substituted alkyl and
- halogen, or

$R_1$ and $R_2$ together with the carbon atoms to which they are attached, represent an optionally substituted 5- or 6-membered ring which may optionally have one or more heteroatoms,
$R_3$ is selected from the group consisting of

- optionally substituted alkyl,
- optionally substituted alkoxy,
- optionally substituted amino and
- hydroxy,

or pharmaceutically acceptable salts thereof for the use in the treatment and prophylaxis of iron deficiency symptoms and iron deficiency anemias.

2. Iron(III)-complex compounds for the use according to claim 1, wherein $R_3$ is optionally substituted amino.

3. Iron(III)-complex compounds for the use according to claim 1 or 2,
wherein
$R_1$ is selected from the group consisting of:

- alkyl, being optionally substituted by 1 to 3 substituents, selected from the group consisting of: hydroxy, alkoxy, alkoxycarbonyl and aminocarbonyl,
- alkoxycarbonyl, being optionally substituted by 1 to 3 substituents, selected from the group consisting of: hydroxy and alkoxy, and
- alkoxy, being optionally substituted by 1 to 3 substituents, selected from the group consisting of: hydroxy, alkoxy and halogen,

$R_2$ is selected from the group consisting of:

- hydrogen,
- alkyl, being optionally substituted by 1 to 3 substituents, selected from the group consisting of: hydroxy, alkoxy, halogen and alkoxycarbonyl,
- halogen, selected from chlorine and fluorine,

or
$R_1$ and $R_2$ together with the carbon atoms to which they are attached, form an optionally substituted 5- or 6-membered ring which may optionally have one or two heteroatoms, and may carry one to three further substituents selected from the group consisting of: hydroxy, alkoxy, alkoxycarbonyl and aminocarbonyl, and
$R_3$ is selected from the group consisting of:

- hydroxy,
- alkyl, being optionally substituted by 1 to 3 substituents, selected from the group consisting of: hydroxy, alkoxy and alkoxycarbonyl,
- alkoxy, being optionally substituted by 1 to 3 substituents, selected from the group consisting of: hydroxy, alkoxy and alkoxycarbonyl, and
- amino, being optionally substituted by 1 to 3 substituents, selected from the group consisting of: alkyl, hydroxyalkyl, alkoxy und alkoxycarbonyl,

or pharmaceutically acceptable salts thereof.

**4.** Iron(III)-complex compounds for the use according to claim 1, 2, or 3, wherein
$R_1$ is selected from the group consisting of:

- methyl
- iso-propyl
- tert.-butyl
- methoxy
- ethoxy
- methoxycarbonyl and
- ethoxycarbonyl,

$R_2$ is selected from the group consisting of:

- hydrogen
- methyl and
- fluorine, or

$R_1$ and $R_2$ together form a propylene ($-CH_2-CH_2-CH_2-$)- or butylene ($-CH_2-CH_2-CH_2-CH_2-$) group, and
$R_3$ is selected from the group consisting of:

- hydroxy
- methoxy
- ethoxy
- amino
- dimethylamino
- 3-propylamino
- 2-ethylamino
- morpholino
- piperidino and
- 4-hydroxypiperidino

or pharmaceutically acceptable salts thereof.

**5.** Iron(III)-complex compounds for the use according to any of claims 1 to 4, having the following formula:

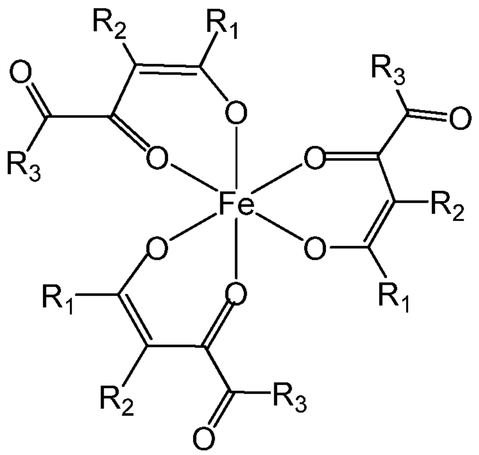
,

wherein $R_1$, $R_2$, and $R_3$ each may be the same or different and are defined as above and pharmaceutically acceptable salts thereof.

6. Iron(III)-complex compounds for the use according to any of claims 1 to 5,
wherein the symptoms associated with iron deficiency conditions and iron deficiency anemias include: fatigue, listlessness, lack of concentration, low cognitive efficiency, difficulties in finding the right words, forgetfulness, unnatural pallor, irritability, acceleration of heart rate (tachycardia), sore or swollen tongue, enlarged spleen, desire for strange foods (pica), headaches, lack of appetite, increased susceptibility to infections, depressive moods.

7. Iron(III)-complex compounds for the use according to any of claims 1 to 6, for the treatment of iron deficiency anemia in pregnant women, latent iron deficiency anemia in children and adolescents, iron deficiency anemia caused by gastrointestinal abnormalities, iron deficiency anemia due to blood loss, such as gastrointestinal hemorrhage (e.g. due to ulcers, carcinoma, hemorrhoids, inflammatory disorders, application of acetylsalicylic acid), iron deficiency anemia caused by menstruation, iron deficiency anemia caused by injuries, iron deficiency anemia due to sprue, iron deficiency anemia due to reduced dietary iron uptake, in particular in selectively eating children and adolescents, immunodeficiency caused by iron deficiency anemia, brain function impairment caused by iron deficiency anemias, restless leg syndrome caused by iron deficiency anemias, iron deficiency anemias in the case of cancer, iron deficiency anemias caused by chemotherapies, iron deficiency anemias triggered by inflammation (AI), iron deficiency anemias in the case of congestive cardiac insufficiency (CHF; congestive heart failure), iron deficiency anemias in the case of chronic renal insufficiency stage 3-5 (CKD 3-5; chronic kidney diseases stage 3-5), iron deficiency anemias triggered by chronic inflammation (ACD), iron deficiency anemias in the case of rheumatoid arthritis (RA), iron deficiency anemias in the case of systemic lupus erythematosus (SLE) and iron deficiency anemias in the case of inflammatory bowel diseases (IBD).

8. Iron(III)-complex compounds for the use according to any of claims 1 to 7, for oral administration.

9. Iron(III)-complex compounds for the use according to claim 8, wherein a solid formulation, such as pills, tablets, enteric-coated tablets, film-coated tablets, layer tablets, sustained release formulations, depot formulations, dragees, suppositories, granules, micro-capsules, micro-formulations, nano-formulations, capsules, enteric-coated capsules and powders, or liquid formulations, including a drinkable formulation such as syrup, elixir, solution, suspension, juice are administered.

10. Medicament, containing the iron(III) complex compounds as defined in any one of claims 1 to 5 and at least one physiologically compatible carrier or excipient.

11. Composition containing the iron(III) complex compounds as defined in any one of claims 1 to 5, in combination with at least one further medicament which acts on the iron metabolism.

## Revendications

1. Composé de coordination 2,4-dioxo-1-carbonyle de fer (III) contenant au moins un ligand de la formule (I) ;

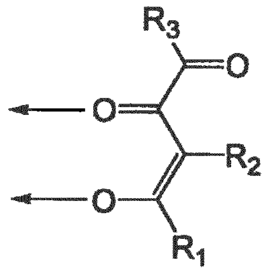

(I)

dans lequel
chacune des flèches représente une liaison coordinatrice à un ou différents atomes de fer, et
$R_1$ est sélectionné parmi le groupe, consistant en

- l'alkyle éventuellement substitué,
- l'alkoxy éventuellement substitué, et
- l'alkoxycarbonyle éventuellement substitué,

$R_2$ est sélectionné parmi le groupe, consistant en

- l'hydrogène,
- l'alkyle éventuellement substitué, et
- l'halogène, ou

$R_1$ et $R_2$ ensemble avec l'atome de carbone auquel ils sont liés, forment un anneau éventuellement substitué à 5 ou 6 membres, qui éventuellement peut avoir un ou plusieurs hétéroatomes,
$R_3$ est sélectionné parmi le groupe, consistant en

- l'alkyle éventuellement substitué,
- l'alkoxy éventuellement substitué,
- l'amino éventuellement substitué, et
- l'hydroxy,

ou les sels pharmaceutiquement acceptables,
pour l'application dans le traitement et la prophylaxie de conditions de carence en fer et d'anémies due à une carence en fer.

**2.** Composé de coordination de fer (III) pour l'application selon la revendication 1, dans lequel $R_3$ est amino éventuellement substitué.

**3.** Composé de coordination de fer (III) selon la revendication 1 ou 2,
dans lequel
$R_1$ est sélectionné parmi le groupe, consistant en

- l'alkyle qui éventuellement peut être substitué par 1 à 3 substituants, qui sont sélectionnés parmi le groupe, qui consiste en : l'hydroxy, l'alkoxy, l'alkoxycarbonyle et l'aminocarbonyle,
- l'alkoxycarbonyle, qui éventuellement peut être substitué par 1 à 3 substituants, qui sont sélectionnés parmi le groupe, qui consiste en : l'hydroxy, et l'alkoxy, et
- l'alkoxy qui éventuellement peut être substitué par 1 à 3 substituants, qui sont sélectionnés parmi le groupe, qui consiste en : l'hydroxy, l'alkoxy et l'halogène,

$R_2$ est sélectionné parmi le groupe, consistant en

- l'hydrogène,
- l'alkyle qui éventuellement peut être substitué par 1 à 3 substituants, qui sont sélectionnés parmi le groupe, qui consiste en : l'hydroxy, l'alkoxy, l'halogène et l'akoxycarbonyle,
- l'halogène, sélectionné parmi le chlore et le fluor,

ou
$R_1$ et $R_2$ ensemble avec l'atome de carbone auquel ils sont liés, forment un anneau à 5 ou 6 membres, qui éventuellement peut avoir un ou deux hétéroatomes, et qui peut porter un à trois autres substituants, qui sont sélectionnés parmi le groupe, consistant en l'hydroxy, l'alkoxy, et l'aminocarbonyle, et
$R_3$ est sélectionné parmi le groupe, consistant en

- l'hydroxy,
- l'alkyle, qui éventuellement peut être substitué par 1 à 3 substituants, qui sont sélectionnés parmi le groupe, qui consiste en : l'hydroxy, l'alkoxy et l'alkoxycarbonyle,
- l'alkoxy qui éventuellement peut être substitué par 1 à 3 substituants, qui sont sélectionnés parmi le groupe, qui consiste en : l'hydroxy, l'alkoxy et l'alkoxycarbonyle, et
- l'amino, qui éventuellement peut être substitué par 1 à 3 substituants, qui sont sélectionnés parmi le groupe, qui consiste en : l'alkyle, l'hydroxyalkyle, l'alkoxy et l'alkoxycarbonyle

ou les sels pharmaceutiquement acceptables de ceux-ci.

**4.** Composé de coordination de fer (III) pour l'application selon la revendication 1, 2 ou 3, dans lequel
$R_1$ est sélectionné parmi le groupe, consistant en

- le méthyle,
- l'isopropyle
- le ter-butyle
- le méthoxy
- l'éthoxy
- le méthoxycarbonyle et
- l'éthoxycarbonyle,

$R_2$ est sélectionné parmi le groupe, consistant en

- l'hydrogène,
- le méthyle et
- le fluor, ou

$R_1$ et $R_2$ ensemble forment un groupe propylène- ($-CH_2-CH_2-CH_2-$) ou butylène- ($-CH_2-CH_2-CH_2-CH_2-$), et
$R_3$ est sélectionné parmi le groupe, consistant en

- l'hydroxy
- le méthoxy
- l'éthoxy
- l'amino
- le diméthylamino
- le 3-propyleamino
- le 2-éthylamino
- le morpholino
- le pipéridino et
- le 4-hydroxypipéridino

ou les sels pharmaceutiquement acceptables de ceux-ci.

**5.** Composé de coordination de fer (III) pour l'application selon l'une des revendications 1 à 4, qui ont la formule suivante :

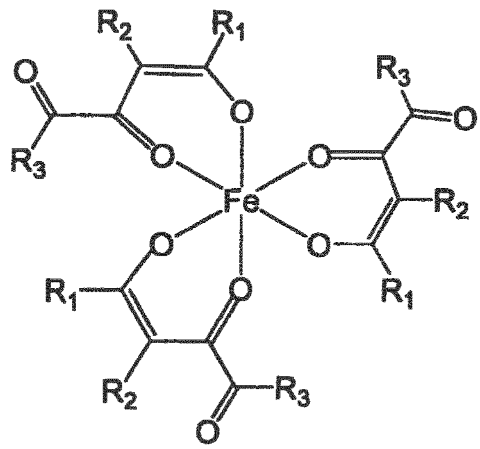

,

dans lequel
chacun de $R_1$, $R_2$ et $R_3$ peut être égal ou différent et sont définis tel que ci-dessus, et les sels pharmaceutiquement acceptables de ceux-ci.

6. Composé de coordination de fer (III) pour l'application selon l'une des revendications 1 à 5, dans lequel les symptômes liés aux conditions de carence en fer et d'anémies due à une carence en fer comprennent : la fatigue, le manque d'initiative, le manque de concentration, l'efficience cognitive faible, des difficultés à trouver les mots justes, l'oubli, la pâleur peu naturelle, l'irritabilité, l'accélération de la fréquence cardiaque (la tachycardie), la langue gonflée et irritée, la rate agrandie, les envies de femmes enceintes (le pica), la céphalée, le manque d'appétit, la susceptibilité accrue aux infections, les sentiments dépressifs.

7. Composé de coordination de fer (III) pour l'application selon l'une des revendications 1 à 6, pour le traitement de l'anémie due à une carence en fer chez les femmes enceintes, de l'anémie latente due à une carence en fer chez les enfants et les adolescents, de l'anémie latente due à une carence en fer due aux anormalités gastro-intestinales, de l'anémie latente due à une carence en fer due à la perte de sang, tel que par des saignements gastro-intestinaux (ex. dus à des ulcères, des carcinomes, des hémorroïdes, des troubles inflammatoires, l'ingestion de l'acide salicylique), de l'anémie due à une carence en fer due à la menstruation, de l'anémie due à une carence en fer due aux lésions, de l'anémie due à une carence en fer due à la psilose (sprue), de l'anémie due à une carence en fer due à l'ingestion de fer réduite dans l'alimentation, surtout chez les enfants mangeant sélectivement et chez les adolescents, de la déficience immunitaire provoquée par une anémie due à une carence en fer, de l'ingérence de la performance cérébrale due aux anémies due à une carence en fer, du syndrome de Restless Leg du aux anémies due à une carence en fer, de aux anémies due à une carence en fer, des anémies due à une carence en fer provoquées par l'inflammation (AI), des anémies due à une carence en fer chez les l'insuffisance cardiaque congestive (ICC, CHF ; congestive heart failure), des anémie due à une carence en fer chez l'insuffisance rénale chronique stade 3-5 (CKD 3-5, chronic kidney diseases stage 3-5), des anémie due à une carence en fer provoquées par l'inflammation chronique (ACD), des anémie due à une carence en fer chez l'arthrite rhumatoïde, (RA ; rhumatoïde arthrites), des anémie due à une carence en fer chez le lupus érythémateux systémique (SLE ; systemic lupus eryrthematosus) et des anémie due à une carence en fer chez des maladies inflammatoires chroniques intestinales (IBD ; inflammatory bowel diseases).

8. Composé de coordination de fer (III) pour l'application selon l'une des revendications 1 à 7 pour l'application orale.

9. Composé de coordination de fer (III) pour l'application selon la revendication 8, dans lequel une formulation solide, tel que les pilules, les comprimés, les comprimés gastro-résistants, les comprimés pelliculés, les comprimés à couche, les formulations retard, les formulations dépôt, les dragées, les suppositoires, le granulat, les microcapsules, les micro-formulations, les nano-formulations, les capsules, les capsules gastro-résistante et les poudres, ou une formulation liquide, incluant une formulation buvable, tel que le sirop, l'élixir, la solution, la suspension, le jus, est appliquée.

10. Médicament contenant des composés de coordination de fer (III) tel que définis selon l'une des revendications 1 à 5 ainsi que pour le moins un porteur pharmaceutiquement acceptable ou excipient, respectivement.

**11.** Composition contenant des composés de coordination de fer (III) tel que définis selon l'une des revendications 1 à 5, en association avec au moins un autre médicament qui agisse sur le métabolisme du fer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20081455586 A
- WO 2007062546 A
- WO 20040437865 A
- US 2003236224 A
- EP 150085 A
- CN 101481404
- EP 939083 A
- JP 02083400 A
- US 474670 A
- CN 1687089
- US 2009035385 A
- EP 308362 A
- ES 2044777
- EP 159194 A
- EP 138420 A
- EP 107458 A
- WO 2006037449 A
- JP 05271157 A
- JP 199270884 B
- JP 04221391 A
- JP 199186727 B
- US 20060134227 A
- DE 83007117
- WO 2005074899 A2
- US 6121287 A
- GB 842637 A


### In der Beschreibung aufgeführte Nicht-Patentliteratur


- **M.W. HENTZE.** Balancing acts: molecular control of mammalian iron metabolism. *Cell,* 2004, vol. 117, 285-297
- *Biometals,* 2009, vol. 22, 701-710
- Novel synthesis of Tris(acetylacetonato)iron (III). **CHAUDHURI MIHIR K et al.** JOURNAL OF THE CHEMICAL SOCIETY. DALTON TRANSACTIONS. ROYAL SOCIETY OF CHEMISTRY, 01. Januar 1983, 839-840
- **LONDON, J. E. ; SMITH, D. M.** *Preliminary toxicological study of ferric acetylacetonate,* 1983
- **M FIALLO.** Metal anthracycline complexes as a new class of anthracycline derivatives. *INORGANICA CHIMICA ACTA,* 01. Juli 1987, vol. 137 (1-2), ISSN 0020-1693, 119-121
- *Bull. Chem. Soc. Jpn.,* 1993, vol. 66, 841-841
- **J. WANG et al.** *Can. J. Chem.,* 2009, vol. 87, 328-334